# EUROPEAN PATENT SPECIFICATION

(11) **EP 2 285 355 B1**
(45) Date of publication and mention of the grant of the patent: **24.01.2018**
(21) Application number: 09742166.3
(22) Date of filing: 08.05.2009
(51) Int. Cl.: A61K 9/20, A61J 3/10, B01J 2/22, B07B 7/00

(54) **PROCESS FOR PREPARING A HIGH DRUG LOAD TABLET**
VERFAHREN ZUR HERSTELLUNG EINER TABLETTE MIT HOHEM WIRKSTOFFGEHALT
PROCÉDÉ POUR PRÉPARER UN COMPRIMÉ À FORTE CHARGE EN SUBSTANCE MÉDICAMENTEUSE

(30) Priority: 09.05.2008 FI 20080352
(43) Date of publication of application: 23.02.2011
(73) Proprietor: Atacama Labs Oy, 00100 Helsinki (FI)
(72) Inventor: POLITI, Giovanni, FI-00310 Helsinki (FI); HEILAKKA, Erkki, FI-00950 Helsikni (FI)
(74) Representative: Teuten, Andrew John
(86) International application number: PCT/EP2009/055628
(87) International publication number: WO 2009/135948

(56) References cited:
- WO-A1-98/35672
- WO-A1-99/11261
- WO-A1-99/25343
- WO-A1-2006/066930
- WO-A1-2009/101258
- WO-A2-2008/056021
- WO-A2-2009/135950
- WO-A2-2009/135951
- US-A1- 2004 126 429

## Description

### TECHNICAL FIELD OF INVENTION

The invention relates to a method for preparing a tablet involving dry granulation as claimed.

### BACKGROUND OF THE INVENTION

Tablets are one of the most frequently employed delivery forms for most medicinal preparations. This situation can be explained by the fact that this dosage form allows for accurate dosage of the active component of the medicinal formulation. Furthermore, handling and packaging are easier and shelf life and stability of these preparations are generally better than those of other formulations.

These same arguments also explain the reason why tablets are often used as media for other applications such as food, including confectionery products, aromas or sweeteners, detergents, dyes or phytosanitary products.

A solid bulk of granulate mass, which is necessary for manufacturing tablets, can be manufactured using two main processes, wet granulation or dry granulation. Tablets may also be manufactured using direct compression. Direct compression relates to the tableting process itself rather than preparation of the starting material.

In wet granulation, components are typically mixed and granulated using a wet binder. The wet granulates are then sieved, dried and optionally ground prior to compressing into tablets. Wet granulation is used extensively in the pharmaceutical industry although it has proven to be a difficult method, mainly because the liquids needed in the granule and tablet manufacturing process often have an adverse effect on the characteristics of the active pharmaceutical ingredients (APIs) and/or on the end product such as a tablet.

Dry granulation is usually described as a method of controlled crushing of precompacted powders densified by either slugging or passing the material between two counter-rotating rolls. More specifically, powdered components that may contain very fine particles are typically mixed prior to being compacted to yield hard slugs which are then ground and sieved before the addition of other ingredients and final compression to form tablets. Because substantially no liquids are used in the dry granulation process, the issues related to wet granulation are avoided. Although dry granulation would in many cases appear to be the best way to produce products such as tablets containing APIs, it has been relatively little used because of the challenges in producing the desired kind of granules as well as managing the granulated material in the manufacturing process. Known dry granulation methods, as well as the known issues related to them are well described in scientific articles, such as the review article "Roll compaction / dry granulation: pharmaceutical applications" written by Peter Kleinebudde and published in European Journal of Pharmaceutics and Biopharmaceutics 58 (2004) at pages 317-326.

Direct compression is generally considered to be the simplest and the most economical process for producing tablets. However, it may only be applied to materials that do not need to be granulated before tableting. Direct compression requires only two principal steps; i.e., the mixing of all the ingredients and the compression of this mixture. However, direct compression is applicable to only a relatively small number of substances as the ingredients of the tablets often need to be processed by some granulation technique to make them compressible and/or for improving their homogeneity and flow-ability.

A component of a tablet is usually described as being either an excipient or an active ingredient. Active ingredients are normally those that trigger a pharmaceutical, chemical or nutritive effect and they are present in the tablet only in the amount necessary to provide the desired effect. Excipients are inert ingredients that are included to facilitate the preparation of the dosage forms or to adapt the release characteristics of the active ingredients, or for other purposes ancillary to those of the active ingredients.

Excipients can be characterized according to their function in the formulation as, for instance, lubricants, glidants, fillers (or diluents), disintegrants, binders, flavors, sweeteners and dyes.

Lubricants are intended to improve the ejection of the compressed tablet from the die of the tablet-making equipment and to prevent sticking in the punches.

Glidants are added to improve the powder flow. They are typically used to help the component mixture to fill the die evenly and uniformly prior to compression.

Fillers are inert ingredients sometimes used as bulking agents in order to decrease the concentration of the active ingredient in the final formulation. Binders in many cases also function as fillers.

Disintegrants may be added to formulations in order to help the tablets disintegrate when they are placed in a liquid environment and so release the active ingredient. The disintegration properties usually are based upon the ability of the disintegrant to swell in the presence of a liquid, such as water or gastric juice. This swelling disrupts the continuity of the tablet structure and thus allows the different components to enter into solution or into suspension
Binders are used to hold together the structure of the tablets. They have the ability to bind together the other ingredients after sufficient compression forces have been applied and they contribute to the integrity of the tablets.

Finding the proper excipients for particular APIs and determining the proper manufacturing process for the combination of excipients and APIs can be a time-consuming job that may lengthen the design process of a pharmaceutical product, such as a tablet significantly, even by years.

Both the dry and wet granulation methods of the prior art may produce solid bridges between particles within granules that may be undesirable for example in that they lead to unsatisfactory subsequent tablet characteristics. The solid bridges may be caused by partial melting, hardening binders or crystallization of dissolved substances. Partial melting may for example occur when high compaction force is used in dry granulation methods. When the pressure in the compaction process is released, crystallization of particles may take place and bind the particles together. Introduction of hardening binders is common in pharmaceutical wet granulations when a binder is included in the granulating solvent. The solvent forms liquid bridges, and the binder will harden or crystallize on drying to form solid bridges between the particles. Examples of binders which can function in this way are polyvinylpyrrolidone, cellulose derivatives (e.g. carboxymethylcellulose) and pregelatinized starch. Substances, e.g. lactose, which can dissolve during a wet granulation process may subsequently crystallize on drying acting as a hardening binder.

Electrostatic forces may also be important in causing powder cohesion and the initial formation of agglomerates, e.g. during mixing. In general they do not contribute significantly to the final strength of the granule. Van der Waals forces, however, may be about four orders of magnitude greater than electrostatic forces and can contribute significantly to the strength of granules, e.g. those produced by dry granulation. The magnitude of these forces increases as the distance between particle surfaces decreases.

In addition to finding a practical manufacturing process for a pharmaceutical product, validation of the manufacturing process is essential. Validation means that the process must be able to reliably produce a consistently acceptable and predictable outcome each time the process is used. Wet granulation methods are quite challenging to manage in this respect. The wet granulation process is often quite vulnerable to small changes in manufacturing conditions. For example, variations in the moisture content of starch in the manufacturing process after drying may produce a tablet that is too hygroscopic or which has a reduced shelf life. When a pharmaceutical product is being developed in laboratory conditions, the conditions can be controlled relatively easily. However, the conditions available in mass production environments are typically less accurately controllable thus making validation of the manufacturing process a difficult and time consuming task. The same can be said about direct compression methods where the quality of the final product depends on the physical properties of the API and excipients. A small change in such properties can result, for example, in segregation and flow-ability problems.

Because of the manufacturing and process validation issues related to wet granulation and direct compression methods, it is desirable, particularly in the pharmaceutical industry, to use dry granulation techniques whenever possible. However, the dry granulation methods known in the prior art produce granules that are seldom usable in a tablet manufacturing process. Conflicting process design parameters often lead to compromises where some qualities of the resulting granule product may be good, but other desirable qualities are lacking or absent. For example, the flow characteristics of the granules may be insufficient, the non-homogeneity of the granules may cause segregation in the manufacturing process or capping in tablets, or some of the granules may exhibit excessive hardness, all of which can make the tableting process very difficult, slow and sometimes impossible. Furthermore, the bulk granules may be difficult to compress into tablets. Alternatively or additionally, the disintegration characteristics of the resulting tablets may be sub-optimal. Such problems commonly relate to the non-homogeneity and granule structure of the granulate mass produced by the compactor. For instance, the mass may have too high a percentage of fine particles or some granules produced by the compactor may be too dense for effective tableting.

It is also well known in the art that in order to get uniform tablets the bulk to be tableted should be homogeneous and should have good flow characteristics.

In prior art dry granulation processes such as roll compaction, the resulting bulk is not generally homogeneously flowing, for example because of the presence of relatively large (1-3 mm) and dense granules together with very small (e.g. 1-30 µm) particles. This can cause segregation as the large, typically dense and/or hard granules of the prior art flow in a different way to the fine particles when the granulate mass is conveyed in the manufacturing process, e.g. during tableting. Because of the segregation, it is often difficult to ensure production of acceptable tablets. For this reason, in the art there are some known devices in which the small particles and sometimes also the biggest particles are separated from the rest of the granules with the help of a fractionating device such as (a set of) vibrating screen(s). This process is generally complicated and noisy and the result is a relatively homogeneously flowing bulk where the granules are hard and difficult to compress into tablets. Furthermore, the process of separating small particles from granules becomes very difficult if the material is sticky and the screen-size is not big enough. Generally in this process the apertures of the screen must have a minimum dimension of at least 500 µm.

Another problem which occurs in dry granulation methods of the prior art is the difficulty of preparing, in the development stage, a pilot bulk which is representative of the production bulk. Thus, the compaction forces and the other compaction parameters used at the laboratory scale can be very different from those used at the production scale. As a result the properties, e.g. flow-ability of the production bulk can be very different from that which has been prepared in a pilot facility. One sieving method applicable in laboratory scale is air sieving. One conventional air sieve involves passing a powder through a mesh of defined size in order to exclude particles below the specified size (the desired granules are retained above the mesh and the rejected particles pass below). Air is passed through the mesh to carry away the fine particles. The problem with the air sieves of the prior art is that their capacity is not sufficient for industrial production of granulate mass. Furthermore, the air sieves that rely on mesh size in the separation of rejected material often exclude desirable small granules from the acceptable granulate mass when separating out the fine particles from the mass. Yet further, fragile granules may break in the sieving process where undersize particles are sucked through the apertures of the sieve.

Patent application WO 99/11261 discloses dry-granulated granules that may comprise API only. In the method disclosed in the application, an air sieve known in the prior art is used for separating fine particles (particles and granules smaller than 150 or 125 µm) from granules comprising up to 100% of API. The sieving utilizes a sieve whose mesh size is about the maximum size of rejectable particles, e.g. 150 µm. It seems that the granules of the disclosure have been created using relatively high compaction forces since the proportion of fine particles (smaller than 125 µm) after compaction is at most around 26 per cent (see table 1). The method results, following sieving, in a flowing homogeneous granulate mass that would be expected to comprise generally hard granules and that substantially is lacking granules and particles smaller than 150 or 125 µm.

U.S. patent US 4,161,516 teaches a composition for treating airway disease using soft tablets or granules for inhalation administration. The method of the patent is suitable for producing granules that are soft enough to break apart in an airstream.

U.S. Patent 6,752,939 teaches a method and an apparatus for predicting the suitability of a substance for dry granulation by roller compaction using small sample sizes.

U.K. Patent 1,558,153 discloses a method for producing organic dye material from finely divided particles by compressing said finely divided particles to produce a coherent mass of material, comminuting said coherent mass of material, and recovering granular material in the particle size range of 100-1000 microns from said comminuted material. The finest particles are removed by air flow.

High drug load in combination with good tensile strength and suitable disintegration profile is often difficult to achieve in a tablet using traditional formulation techniques.

Some binder excipients are known to maintain their tablettability the better, the less the material is compacted in the granulation process. However, lightly compacted granules may be very fragile and the bulk may contain a large proportion of fine particles. Sieving techniques used in traditional dry granulation processes are not generally suitable for producing well-flowing granular mass from such material as the sieves may clog and the fragile granules may be destroyed in the sieving process.

We have now found an improved method and apparatus for dry granulation which is highly suitable for preparing tablets having a high API content, high tensile strength and a suitable disintegration profile. The method may be applicable to a wide variety of APIs.

### BRIEF DESCRIPTION OF THE INVENTION

Specifically, we have now found a dry granulation method as claimed for making tablets having a drug load of at least 50% and less than 90% utilizing a dry granulation method that produces well-flowing granules from various materials, including APIs and excipients and their combinations.

A compaction force is used to produce granules from material that comprises binder excipient and optionally at least one active ingredient and one or more other excipients. Suitably the compaction force for compacting binder excipient is low to preserve a high level of binding capabilities of the binder for the tableting phase.

An aspect of the invention is a dry-granulation method for producing a tablet comprising (a) pharmaceutical active ingredient in an amount 50-90% w/w, e.g. at least 50%, 51%, 60% or 70% and less than 80%, 89% or 90% w/w such as 51-89% w/w, and (b) one or more excipients in an amount 10-50% w/w, e.g. at least 10%, 11 % or 20% and less than 30%, 40%, 49% or 50% w/w such as 11-49% w/w, including at least a binder which comprises (i) preparing granules from a powder comprising a binder, a pharmaceutically active ingredient and optionally one or more other excipients or pharmaceutical active ingredients by a process in which a compaction force is applied to the powder to produce a compacted mass comprising a mixture of fine particles and granules and separating and removing fine particles and/or small granules from the granules by means of an apparatus comprising fractionating means wherein the fractionating means comprises a rotating device along the axis of which the compacted mass is moved in a gas stream through said rotating device, wherein the direction of the flow of the gas stream has a component which is contrary to that of the direction of flow of the compacted mass, wherein the rotating device comprises one or more apertures through which the gas stream flows out of the device and through which the fine particles and/or small granules are entrained and collecting the accepted granules; (ii) blending the accepted granules with other components of the tablet in granular or fine powder form wherein in step (ii) at least one other component of the tablet formulation is in granular form and is prepared from a powder comprising said other component by a process in which a compaction force is applied to the powder to produce a compacted mass comprising a mixture of fine particles and granules and separating and removing fine particles and/or small granules from the granules by means of an apparatus comprising fractionating means wherein the fractionating means comprises a rotating device along the axis of which the compacted mass is moved in a gas stream through said rotating device, wherein the direction of the flow of the gas stream has a component which is contrary to that of the direction of flow of the compacted mass, wherein the rotating device comprises one or more apertures through which the gas stream flows out of the device and through which the fine particles and/or small granules are entrained; and (iii) compressing the resultant blend to form a tablet; with the proviso that the tablet does not comprise (a) paracetamol, maize starch and microcrystalline cellulose in a ratio of 60:20:20 w/w, (b) acebutolol HCl and starch in a ratio of 90:10 w/w, (c) sodium valproate, hypromellose and maize starch in a ratio of 90:5:5 w/w, (d) ketoprofen and maize starch in a ratio of 50:50 w/w or (e) metformin HCl, microcrystalline cellulose and maize starch in a ratio of 80:14:6 w/w

The granules of step (i) may be prepared from a powder also comprising a disintegrant.

The granules of step (i) may also be prepared from a powder also containing an active ingredient.

Suitably the binder content of the powder of step (i) is 5% or more eg 6% or more e.g. 10% or more w/w.

In some embodiments, granules may be prepared in step (i) from a powder comprising all the components of the tablet formulation except lubricant and in step (ii) the accepted granules are blended with lubricant.

In some embodiments, at least one other component of the tablet formulation may be in granular form in step (ii) and is prepared from a powder comprising another component by a process characterized in that a compaction force is applied to the powder to produce a compacted mass comprising a mixture of fine particles and granules and separating and removing fine particles and/or small granules from the granules by entraining the fine particles and/or small granules in a gas stream. The other component of the tablet formulation may also comprise an active ingredient.

The ratio of binder to disintegrant may be e.g. between 10:1 and 1:1 w/w, suitably around 4:1 w/w.

In some embodiments, the active ingredient may be e.g. moisture sensitive, heat sensitive or insoluble in water.

Another aspect of the invention is a tablet obtained according to the method of any embodiment of the invention.

Yet another aspect of the disclosure is a method for preparing granules from a powder comprising (a) active ingredient in an amount of 50-90% e.g. at least 50%, 51%, 60% or 70% and less than 80%, 89%or 90% w/w such as 51-89% w/w and (b) one or more excipients including at least a binder in an amount of 10-50% w/w e.g. at least 10%, 11% or 20% and less than 30%, 40%, 49% or 50% w/w such as 11-49% w/w by a process characterized in that a compaction force is applied to the powder to produce a compacted mass comprising a mixture of fine particles and granules and separating and removing fine particles and/or small granules from the granules by entraining the fine particles and/or small granules in a gas stream wherein the powder does not comprise (a) paracetamol, maize starch and microcrystalline cellulose in a ratio of 60:20:20 w/w, (b) acebutolol HCl and starch in a ratio of 90:10 w/w, (c) sodium valproate, hypromellose and maize starch in a ratio of 90:5:5 w/w or (d) ketoprofen and maize starch in a ratio of 50:50 w/w.

Still yet another aspect of the disclosure is a dry granulate mass obtainable according to the method of preparing granules.

The binder may for example be selected from synthetic polymers such as crospovidone, saccharides such as sucrose, glucose, lactose and fructose, sugar alcohols such as mannitol, xylitol, maltitol, erythritol, sorbitol, water-soluble polysaccharides such as celluloses such as crystalline cellulose, microcrystalline cellulose, powdered cellulose, hydroxypropylcellulose and methyl cellulose, starches, synthetic polymers such as polyvinylpyrrolidone, inorganic compounds such as calcium carbonate and others classified as binders in Arthur H. Kibbe: Handbook of Pharmaceutical Excipients, 3rd Edition, and one, two or more of them in combination.

Suitably, the binder is microcrystalline cellulose.

The disintegrant may for example be selected from carboxymethyl cellulose, Nymcel™, sodium carboxymethyl cellulose, croscarmellose sodium, cellulose such as low substitution degree hydroxypropylcellulose, starch such as sodium carboxymethyl starch, hydroxypropyl starch, rice starch, wheat starch, potato starch, maize starch, partly pregelatinized starch and others classified as disintegrants in Arthur H. Kibbe: Handbook of Pharmaceutical Excipients, 3^{rd} Edition, and one, two or more of them in combination.

Suitably, the disintegrant is starch (e.g. maize starch) or carboxymethylcellulose.

The ratio of binder to distintegrant may be e.g. between 10:1 and 1:1 w/w, suitably around 4:1.

In some embodiments, the granules of step (i) or step (ii) may comprise more than 1, 2 or 5 per cent and up to 30, 20 or 10 per cent (weight) of e.g. metholose or hypromellose (hydroxypropyl methylcellulose) to control the disintegration and dissolution time of the tablet. The dissolution time of such tablet may be e.g. at least 0.5, 1, 4 or 8 hours in the gastric system.

A lubricant may, for example, be selected from magnesium stearate and hydrogenated plant or vegetable oil eg hydrogenated cottonseed oil or any other suitable lubricant known to a person skilled in the art.

In tablets of the invention a relatively low amount of lubricant eg 0.1-5% eg 0.1-0.5% w/w may be employed.

A further aspect of the invention is a tablet obtainable according to the method disclosed herein.

The active ingredient(s) may be sensitive to moisture (e.g. ambient humidity), heat or compression forces. Alternatively or additionally, the active ingredient may be insoluble in water.

As explained below, the method may typically be run as a continuous process.

Suitably the process is carried out in the substantial absence of liquid.

The powder, e.g. the APIs and/or excipients usable in pharmaceutical industry, to be used in the granulation process of the invention, generally comprises fine particles. Further, the powder may typically have a mean particle size of less than 100, 50 or 20 µm. The fine particles in the powder may typically have a minimum particle size of 2, 5 or 10 µm and maximum size of 150, 100 or 75 µm. The inventors believe that the inventive ideas of the method disclosed herein may be applicable to form granules also from powder whose minimum particle size is smaller than the typical minimum size mentioned above, e.g. 0.001, 0.01 or 1 µm.

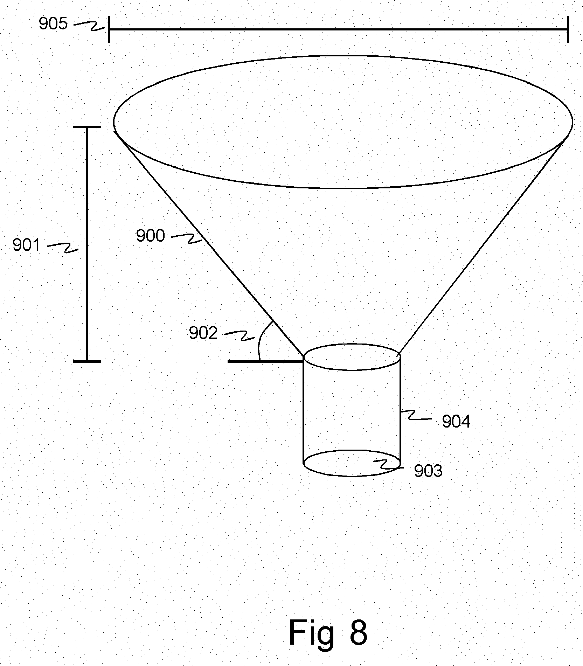

The mean particle size may be measured for example using a set of sieves. In case of very fine powders, also microscopy may be used for analyzing the particle sizes. The flowability of such powders is generally insufficient for e.g. tableting purposes. An exemplary method for determining sufficient flowability of a mass is disclosed in the detailed description of figure 8.

Hence "fine particles" or "fines" are individual particles typically having a mean particle size of less than 100, 50 or 20 µm and a maximum size of 150, 100 or 75 µm.

When several fine particles (e.g. 3, 5, 10 or more) agglomerate to form granules of maximum size of 150, 100 or 75 µm, they are referred to as small granules. Granules larger than the maximum size are referred to as "acceptable granules". Those granules that remain after fine particles and/or small granules have been entrained by the gas stream, are called "accepted granules".

The method will typically further comprise the step of collecting the accepted granules.

The applied compaction force may produce e.g. a compacted ribbon or slug, typically a ribbon. In some embodiments, the thickness of the ribbon or slug may be e.g. at least 1.5, 2 or 3 times the mean diameter of accepted granules. In some embodiments, the thickness of the ribbon may be at least 1, 1.5, 2 or 3 millimeters. The ribbon or slug may then be comminuted into granules. The thickness of the ribbon or slug may have an effect on the properties of the granules produced by the method of the present invention.

The ribbon may comprise strongly compacted and weakly compacted powder. In some embodiments, separate compacting means may be used for producing strongly compacted and weakly compacted powder.

The minimum, optimal and maximum compaction force applicable to the powder may be dependent on the powder material.

The minimum compaction force may be adjusted to a level high enough to prevent degradation of granule properties, e.g. flowability, during storage.

Suitably the compaction force may be provided for example using a roller compactor. Alternatively it may be provided using slugging device. Other compaction methods will be known to the skilled person. The roller compactor or slugging device may be accompanied by an optional flake crushing screen or other device, e.g. oscillating or rotating mill, suitable for producing granules from the compacted material. The optional step of employing a flake crushing screen or other device, will, if necessary, prepare the material for separation of fine particles and/or small granules from other granules.

Thus typically the compaction force is applied to the powder by a process comprising use of a roller compactor to generate a ribbon of compacted powder which is broken up to produce granules e.g. by means of a flake crusher. The flake crusher or similar device may permit the upper size of granules to be controlled e.g. by passing them through a screen. The aperture size of the flake crushing screen may be e.g. 0.5mm, 1.0mm or 1.2mm.

The compaction force may be adjusted to be at minimum such that at least one, five, ten orfifteen per cent of the powder substance becomes acceptable granules during compaction and/or fractionating steps, while the rest of the material remains fine particles and/or small granules.

Suitably the compaction force is a low compaction force.

If the compaction force used is too low, inventors have observed that the granules accepted by the process may be too fragile for e.g. tableting purposes. Such granules may also be too large, e.g. larger than 3 mm. Fragile granules may not flow well enough or be strong enough to be handled e.g. in a tableting process. Too fragile granules may also lose at least some of their flowability over time.

The maximum compaction force may be adjusted so that 75 per cent or less, 70 per cent or less, 65 per cent or less, 50 per cent or less or 40 per cent or less of the powder is compacted into acceptable granules and the rest remains as fine particles and/or small granules. The maximum compaction force is typically up to 500%, 250% or 150% of a minimum compaction force.

For example the mean particle size of the powder may be less than Y µm and the compaction force may be sufficiently low that 75% or less by weight of the powder is compacted into acceptable granules having particle size larger than 1.5 x Y µm and at least 150 µm and the rest remains as fine particles and/or small granules. For instance the average particle size of the powder may be between 1 and 100 µm and the compaction force is sufficiently low that 75% or less by weight of the powder is compacted into acceptable granules having particle size larger than 150µm (and/or a mean size of 100µm or greater) and the rest remains as fine particles and/or small granules.

The mean particle size may be determined e.g. by dividing the bulk into a plurality of fractions using a set of sieves and weighing each of the fractions. Such measuring methods are well known to a person skilled in the art.

When the compaction force is applied by a roller compactor, the compaction force may be such that the ribbon produced by the roller compactor has a tensile strength of around 40-250N i.e. at least 40N, 50N or 60N and less than 250N, 200N or 150N when the thickness of the ribbon is about 4mm. The area of the measured ribbon may be e.g. 3cm x 3cm. The tensile strength of the ribbon may be measured e.g. using device of make MECMESIN™ (Mecmesin Limited, West Sussex, UK) and model BFG200N.

The compaction force may also be such that the bulk volume of the powder is reduced by around 7-40% i.e. at least 7%, 10% or 13% and less than 40%, 35% or 30% following compaction.

The maximum and minimum compaction forces will of course depend on the particular compactor and powder used. Thus, for example the minimum compaction force may be adjusted so that it is the minimum possible compaction force, 15 kN, 20 kN or 30 kN in a Hosokawa™ (Osaka, Japan) Bepex Pharmapaktor L200/50P roller compactor. The maximum compaction force may also be adjusted so that it is 80kN or less, 70kN or less, 60 kN or less or 45 kN or less in a Hosokawa™ Bepex Pharmapaktor L200/50P roller compactor.

Typically a suitable compaction force is 60kN or less e.g. 45kN or less. Typically, a suitable compaction force is 12kN or more eg 16kN or more in a Hosokawa™ Bepex Pharmapaktor L200/50P compactor or equivalent.

The maximum compaction force may also be adjusted so that substantially no solid bridges are formed in the granules of the resulting mass e.g. due to heating of the mass. Some compactors known in the art provide means for cooling the compacted material to alleviate the heating issues introduced by use of high compaction forces. With the method and system of the present invention, this precaution is unnecessary.

The compaction force may be adjusted using a method appropriate for the compactor employed, for example by control of the rate of feed into the compactor.

The above mentioned compaction forces are low and, as explained elsewhere herein, granulate mass compacted using such low forces and processed according to the invention appears to retain good properties of compressibility into tablets. This remark appears to be especially true when the granulate mass comprises a binder.

The gas stream may be provided by any suitable means, e.g. a generator of negative pressure i.e. a vacuum pump such as a suction fan. The gas stream, e.g. air, may be directed through a fractionating chamber. The gas stream separates at least some fine particles and/or small granules from the mass comprising acceptable granules, small granules and fine particles. The separated fine particles and/or small granules entrained in the gas stream may be transferred from the fractionating chamber to a separating device, e.g. a cyclone where the carrier gas is separated from the fine particles and/or small granules. The fine particles and/or small granules are then returned to the system for immediate re-processing (i.e. they are re-circulated for compaction) or they may be placed into a container for later re-processing.

For suitable protection of the system and environment, suitably the gas inlet of the vacuum pump is provided with a receiver filter to trap any particles that, if unfiltered, may pass through the pump. Most suitably the gas inlet of the vacuum pump is provided with a second filter (safety filter) in series with the receiver filter.

Thus, conveniently, fine particles and/or small granules are separated from the acceptable granules by means of an apparatus comprising fractionating means. Desirably, the fractionating means comprises a fractionating chamber.

As discussed in greater detail in the examples, the largest acceptable granules exiting from the fractionating chamber are usually larger in size than the largest granules entering the fractionating chamber. The inventors believe that a process whereby small granules and/or fine particles agglomerate with larger granules takes place during the conveyance of the material through the fractionating chamber.

The direction of the flow of the gas stream has a component which is contrary to that of the direction of flow of the compacted mass in general and accepted granules especially. Typically the direction of the flow of the gas stream is substantially contrary to (e.g. around 150-180° to), and preferably contrary to that of the direction of flow of the compacted mass.

The gas may, for example, be air (suitably dry air). In some embodiments, the gas may contain a reduced proportion of oxygen. In some embodiments, the gas may be e.g. nitrogen.

Suitably the carrier gas is re-circulated in the process. This is especially beneficial for economic reasons when the carrier gas is not air. The fractionating means is also dynamic, i.e. the fractionating means comprises some moving parts.

In a method according to the invention fine particles and/or small granules are separated and removed from the granules by means of an apparatus comprising two or more (eg two) fractionating means in series. In some embodiments, the arrangement may comprise a plurality of static and/or dynamic fractionating means that may be arranged in parallel or in series. In one embodiment, a dynamic fractionating means may be connected in series to a static fractionating means.

The fractionating means comprises means to guide a gas stream into the fractionating means, means to put the compacted mass into motion and means to guide removed fine particles and/or small granules entrained in the gas stream from the fractionating means, e.g. for re-processing. The compacted mass may be put into motion simply by the effect of gravitation and/or by mechanical means.

Advantageously the fractionating means does not require passage of the compacted mass through any sieve (such as a mesh screen). Sieves have a tendency to break up lightly compacted granules, therefore avoidance of use of a sieve permits lightly compacted granules, with their favorable properties, to be preserved e.g. for tableting. Moreover sieves are easily clogged, which disrupts the process, especially when run in continuous operation. Additionally, the eye size of a sieve may vary during the period of operation due to transient clogging.

The fractionating means comprises a rotating device, such as a cylinder (or cone), along the axis of which the compacted mass is moved in the gas stream through said rotating device. Movement of the compacted mass may be by gravitational means or it may be facilitated by mechanical means, or by features of the device (e.g. cylinder). The rotating device may comprise at least one structure for guiding the compacted mass inside the rotating device, such as by provision of a spiral structure. The spiral structure may be formed of channels or baffles which guide the movement of the compacted mass. A component of gravitational assistance or resistance may be provided by tilting the axis of the rotating device. Suitably the compacted mass moves along a helical path within the device. This is advantageous since it increases the path length of the compacted mass and thereby the residence time in the device, and this is expected to increase the efficiency of fractionation. Suitably the length of the helical path is at least twice the linear length of travel along the axis of the device, e.g. at least 2, 3 or 5 times. Suitably there is also at least some movement of the compacted mass relative to the device itself which may thereby create some friction between the mass and the wall of the device. The friction may contribute to the triboelectrification phenomenon that may occur in the fractionating device.

The fractionating means may contain one or more apertures through which fine particles and/or small granules are entrained. In one specific embodiment of the invention the gas stream enters the rotating device along its axis (in the opposite sense to movement of the compacted mass) and exits the rotating device through one or more apertures (perforations) in the side walls of the rotating device. One aperture is the minimum, however two or more (eg 4, 8, 12 or more) may be suitable.

As noted above, the fractionating means comprises a rotating device to move the compacted mass in the fractionating means. The device comprises one or more apertures through which the gas stream flows into and out of the device and through which the fine particles and/or small granules are entrained. The apertures through which gas flows out of the device may be substantially larger than rejectable fine particles, e.g. at least 50%, 100% or 150% of the average diameter of accepted granules. In absolute terms, the apertures may for example have a minimum dimension of around 250 µm, 500 µm or 750 µm or more. This helps prevent the apertures from clogging even when relatively high volumes of fine particles of possibly sticky material need to be separated from the compacted mass. In this sense, the device significantly differs from an air sieve of the prior art where the sieve mesh size must be of about the same size as the largest rejected particle. Instead of relying on the mesh size in the sieving, the fractionating device of the claimed invention relies on the gas stream's ability to entrain fine particles from the moving compacted mass. The determination of the size of acceptable granules is achieved by balancing their gravitational force (together with other forces, e.g. mechanical and centrifugal forces) against the force of the gas stream.

Suitably the fractionating means is provided with means to prevent clogging or build-up. For example it may be provided with a vibrating or ultrasound emitting means. Alternatively since the fractionating means contains apertures (ie in the case of a rotating cylinder with one or more apertures) said apertures may be unclogged by blowing pressurized gas eg air through or across the apertures.

Some of the fine particles and/or small granules may be agglomerated to other granules in the fractionating means by means of the individual or combined influence of the carrier gas stream, mechanical forces, attractive forces and electrostatic forces, for example. Thus, the process may produce granules that are larger than what is produced by the flake crushing screen of the system. In some embodiments, the degree of agglomeration of the compacted mass in the fractionating phase may be significant.

The movement of the mass in the gas stream may be achieved by applying, for example, a mechanical force, gravitational force, centrifugal force or a combination of these. In some embodiments, a mechanically moving component in the fractionating means may not be needed at all to realize the benefits of the present invention. In some embodiments, the acceptable granules fall in a gas stream e.g. by effect of gravitation force and unacceptable particles and granules are moved to at least partially opposite direction by the gas stream. Suitably, however, the compacted mass is moved in the gas stream by means including mechanical means. Typically the average residence time of the compacted mass within the fractionating means is at least 2 seconds, perhaps even at least 5 seconds, although the desired fractionating effect (including any agglomerating effect) may be achievable also in a time frame shorter than that. Residence time may be extended e.g. by providing a helical path.

It should also be noted that the rejected fraction of the mass may also contain acceptable granules. By allowing some recycling of acceptable granules the overall apparatus may be made more efficient and easier to maintain as clogging of fractionating device may be more easily avoided. These rejected acceptable granules may be conveyed to the beginning of the granulating process along with the other rejected material for reprocessing. For efficiency, we prefer that at maximum 30, 45, 60 or 75 per cent of acceptable granules are re-cycled with the fines. The inventors have not observed any detrimental effect on the granulate mass caused by recycling. This is attributable to the use of low compaction force.

The apparatus for use in a method according to the invention may also comprise process monitoring and/or controlling means. For example, the amount of material (typically the weight of material) being in circulation in various components of the apparatus may be monitored and/or controlled by such means.

In order to keep the device in balance for continuous operation, suitably the amount (weight) of powder being conveyed to the compaction means will be measured and controlled as will the amount (weight) of accepted granules being collected and optionally also the amount (weight) of compacted mass leaving the compactor prior to entering the fractionating device. Means of measurement and control include provision of scales to measure weight at various points in the system (eg at the reservoir of powder, the collector of accepted granules and optionally after the compactor and prior to the fractionating device).

In general terms it is desirable: (a) to control the amount of material in the apparatus, (b) to measure flowability of accepted granules, (c) to measure output rate of accepted granules and/or (d) to monitor progress of material in the fractionating means (e.g. by means of provision of a window therein).

Suitably control means are provided to keep the gas flow as steady as possible, especially when one and the same gas stream is used for fractionating as for pneumatic transport.

In some embodiments, the gas flow in the fractionating chamber may be arranged to be an at least partially turbulent flow. In some other embodiments, the gas flow in the fractionating chamber may be arranged to form a laminar flow, e.g. a vortex. In some embodiments, some of the gas flow may be turbulent and some may be laminar.

In the method and apparatus according to the claimed invention, pneumatic transport may be used.

Suitably, the gas used to entrain the fine particles in the compacted mass is in fluid communication with the carrier gas used to convey materials in continuous operation. In some embodiments, different gas streams may be used for fractionation and conveying. Suitably the gas stream employed in the pneumatic conveyor is the same gas stream as is used to entrain the fine particles and/or small granules. In yet further embodiments, conveying of the material may be implemented using some mechanical conveying means , e.g. screw or belt conveyor while fractionation means utilize some suitable gas stream. Construction of such embodiments following teachings of this disclosure is obvious to a person skilled in the art.

Thus, suitably the powder for compaction is conveyed from a reservoir to the means to apply compaction force by means comprising use of a pneumatic conveyor.

The pneumatic transport may use a device, e.g. a cyclone, for separating carrier gas from fine particles. The device may be for example capable of continuous operation at an about even gas flow rate, in the sense that the carrier gas stream used in the fractionating process is not disturbed by pressure changes, e.g. by pressure shocks, such as are required to keep filters of various types open.

"Continuous operation" in this context means ability to operate without maintenance or other interruptions for at least one hour, eight hours or 24 hours.

The carrier gas stream(s) used in the fractionating process and/or the pneumatic conveyance (which suitably are one and the same gas stream) are suitably created by a generator of negative pressure (e.g. a vacuum pump) which draws gas in from another part of the system, typically at the outlet to the fractionating means. A suction fan is a typical example of a vacuum pump. The vacuum pump is suitably provided with at least one filter to capture any particles that may be drawn through the pump. Most suitably two filters are provided in series (i.e. a receiver filter and a safety filter).

Without being limited by theory, the inventors believe that the granulate mass product produced according to the invention is influenced by triboelectric effects caused by passage of powder through the system. It is suggested in prior art that small particles may have a tendency to develop a negative charge whereas larger particles develop a positive charge (or at least a less negative charge) (see e.g. article "Generation of bipolar electric fields during industrial handling of powders" by Ion. I. Inculet et al, Chemical Engineering Science 61 (2006), pages 2249-2253) e.g. when conveyed by a gas stream or otherwise moved in a gas stream. Hence at least some and possibly most of the granules of the dry granule mass appear to comprise a compressed core containing fine particles of material associated by Van der Waals forces and a coating layer containing fine particles and/or small granules of said material associated with said compressed core by electrostatic forces. The inventors have also discovered that, at least in some cases, e.g. with powder containing binder excipient, if granules obtained by the process of the invention are taken and a proportion of the starting material composed of fine particles is added back (eg up to 15% fine particles is added back to a granulate mass that may already have e.g. 20% of fine particles and/or small granules, e.g. mass of "flowability example 3") then the homogeneity, flowability and tabletability of the granulate mass is not adversely affected in a significant manner. The added fines are, perhaps, taken into the porous surface of granules formed by the process of the invention. Inventors thus believe that in some embodiments, it may be possible to use granules of some embodiments of the invention as "carrier granules" that may absorb e.g. into the pores of the granules up to 10%, 20%, 30% or more of fine particles and/or small granules comprising same or different material as the carrier granules. The flowability of such mixture may be at an excellent, very good or good level.

Granulate mass produced according to the invention is believed to have good compressibility because at least the surface of the granules is porous. The compressibility of the granulate mass of the invention may be good, i.e. it may have a Hausner ratio of greater than 1.15, 1.20 or 1.25. The compaction force of the present invention may be adjusted so that the compressibility as indicated by the Hausner ratio stays at good level.

The Hausner ratio may be calculated using formula pₜₐₚ/p_{bulk} where pₜₐₚ represents tapped bulk density of the granulate mass and p_{bulk} represents the loose bulk density of the granulate mass. The bulk densities may be measured by pouring 50 mg of granulate mass into a glass cylinder (e.g. make FORTUNA, model 250:2 ml) having an inner diameter of 3.8mm. After pouring the mass into the cylinder, the volume of the mass is observed from the scale of the glass cylinder and loose bulk density of the mass is calculated. To measure the tapped bulk density, the glass cylinder is tapped 100 times against a table top using a force comparable to a drop from the height of 5 cm. The volume of the tapped mass is observed from the scale of the glass cylinder and tapped bulk density of the mass is calculated.

Surprisingly, and contrary to what is taught in the prior art, e.g. in WO99/11261, the compressibility of the granulate mass of the invention does not generally exhibit any negative influence on the flowability of the granulate mass. For example, a granulate mass of an embodiment of the invention with Hausner ratio above 1.25 generally exhibits very good or excellent flow characteristics.

Porous, well-flowing granules are generally desired in the pharmaceutical industry for example because it is possible to produce enhanced tablets from porous granules. Such tablets may for example disintegrate substantially quicker than tablets manufactured from dense granules. Further, tablets compressed from porous granules often show higher tensile strength than tablets compressed from dense granules. High tensile strength is often desirable for tablets as such tablets are easier to package and transport than fragile tablets.

The granulate mass may be tabletable so that using standard tableting techniques, e.g. using tableting forces available in widely used tableting machines, it is possible to form it into tablets having tensile strength of at least 5N, 10N or 15N. Tensile strength may be measured for example using a measuring device of make MECMESIN™ (Mecmesin Limited, West Sussex, UK) and model BFG200N.

The granules of the invention may be especially useful in preparing multilayer tablets. In multilayer tablets it seems that it is advantageous to use porous granules, as may be prepared according to the process of the invention, to prepare the layers, especially the inner layers. This may facilitate adherence of the layers to each other and particularly adherence of the outer layers to the inner layers. Use of larger size granules, e.g. of size greater than 200 micron or even greater than 400 or 500 micron can also facilitate adherence of layers to each other since it results in a less smooth surface after compression. Multilayer tablets may typically be prepared by first compressing the layers individually and then compressing the layers together. Granules of the invention could be used in all the layers or just some of the layers (e.g. the outer layers).

The granulation method of the invention can be applied for many purposes in the pharmaceutical industry. The method and apparatus use compaction force, suitably low compaction force, and gas stream to form granules of desired properties. The compaction force may be adjusted so that introduction of solid bridges is substantially avoided in the compaction step. The method and apparatus are adapted to treat the product granules gently to avoid breaking them, to separate fine particles and/or small granules from the acceptable granules, and optionally to re-circulate the rejected material for re-processing in the system. The apparatus and method can be made easily adjustable, controllable and more or less continuously operable.

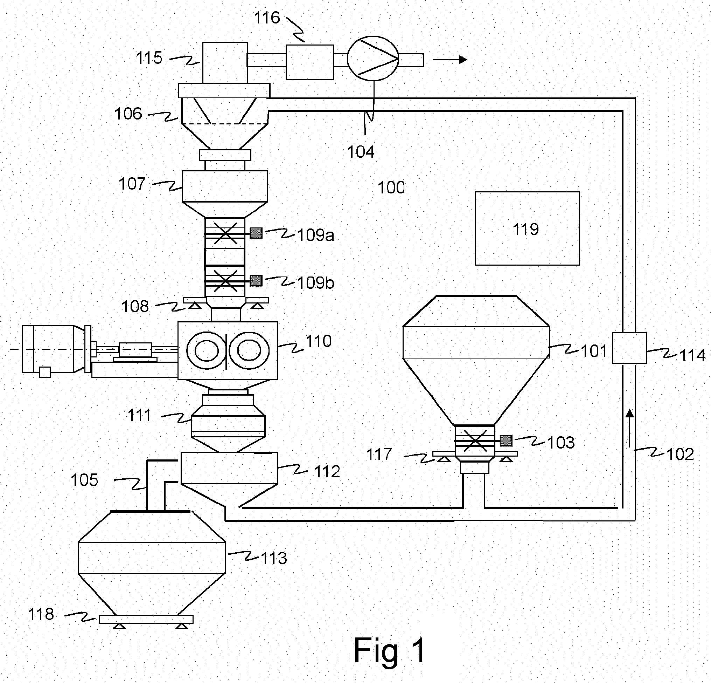

The size distribution and/or flowability of the granules produced by the apparatus may be analyzed in real-time and the size distribution of the granules may be adjusted based on the analysis. For example, the flake crushing screen (see figure 1 below) may be such that the aperture size of the mesh used for flake crushing can be varied by using some adjustment means. Another adjustable parameter typically is the gas flow rate of the fractionating device.

The method can be made economic as it allows re-processing of rejected material with practically no waste, and can be adapted to provide fast treatment of large amounts of material. The apparatus of the present invention may be adapted to be easy to clean and assemble and the process may be adapted to be stable and predictable thus making it easy to control.

Because of, for example, the homogeneity and/or flowability of the resulting granules, issues related to segregation can be avoided. The method of the present invention can be used in both small and large scale applications. Thus, when a product, e.g. granules or a tablet containing API(s) has been successfully developed under laboratory conditions, the time required to set up a validated large-scale manufacturing process can be short.

We have found that the method of the present invention may be used for producing granules of large variety of powder substances usable in pharmaceutical industry.

The method of the present invention may thus be applicable to producing tablets of the invention from material comprising APIs of one or multiple classes of APIs, the classes including for example antipyretics, analgesics, antiphlogistics, hypnosedatives, antihypnotics, antacids, digestion aids, cardiotonics, antiarrhythmics, antihypertensives, vasodilators, diuretics, antiulcers, antiflatulents, therapeutic agents for osteoporosis, antitussives, expectorants, antiasthmatics, antifungals, micturition improvers, revitalizers, vitamins and other orally administered agents. APIs can be used singly or two or more of them can be used in combination.

The method of the present invention may also be applicable to producing tablets of the invention from material comprising specific APIs, for example paracetamol, acebutolol, metformin, fluoxetine, aspirin, aspirin aluminum, acetaminophen, ethenzamide, sazapirin, salicylamide, lactyl phenetidine, isothipendyl, diphenylpyraline, diphenhydramine, difeterol, triprolidine, tripelennamine, thonzylamine, fenethazine, methdilazine, diphenhydramine e.g. as salicylate, carbinoxamine diphenyldisulfonate, alimemazine e.g. as tartrate, diphenhydramine e.g. as tannate, diphenylpyraline e.g. as teoclate, mebhydrolin e.g. as napadisylate, promethazine methylene disalicylate, carbinoxamine e.g. as maleate, chlorophenylamine e.g. as di-maleate, chlorophenylamine e.g. as d-maleate, difeterol e.g. as phosphate, alloclamide, cloperastine, pentoxyverine (carbetapentane), tipepidine, dextromethorphan e.g. as hydrobromide, dextromethorphan phenolphthalinate, tipepidine hibenzate, cloperastine fendizoate, codeine e.g. as phosphate, dihydrocodeine e.g. as phosphate, noscapine, dl-methylephedrine e.g. as saccharin salt, potassium guaiacolsulfonate, guaifenesin, caffeine, anhydrous caffeine, vitamin B1 and derivatives thereof, vitamin B2 and derivatives thereof, vitamin C and derivatives thereof, hesperidin and derivatives thereof and salts thereof, vitamin B6 and derivatives thereof and, nicotinamide, calcium pantothenate, aminoacetate, magnesium silicate, synthetic aluminum silicate, synthetic hydrotalcite, magnesium oxide, aluminum glycinate, coprecipitation product of aluminum hydroxide/hydrogen carbonate, coprecipitation product of aluminum hydroxide/calcium carbonate/magnesium carbonate, coprecipitation product of magnesium hydroxide/potassium aluminum sulfate, magnesium carbonate, magnesium aluminometasilicate, ranitidine, cimetidine, famotidine, naproxen, diclofenac, piroxicam, azulene, indometacin, ketoprofen, ibuprofen, difenidol, promethazine, meclizine, dimenhydrinate, fenethazine e.g. as tannate, diphenhydramine e.g. as fumarate, scopolamine e.g. as hydrobromide, oxyphencyclimine, dicyclomine, metixene, atropine methylbromide, anisotropine methylbromide, scopolamine methylbromide, methylbenactyzium e.g. as bromide, belladonna extract, isopropamide e.g. as iodide, papaverine, aminobenzoic acid, cesium oxalate, aminophylline, diprophylline, theophylline, isosorbide e.g. as dinitrate, ephedrine, cefalexin, ampicillin, sucralfate, allylisopropylacetylurea, bromovalerylurea, and where appropriate (other) pharmaceutically acceptable acid or base addition salts thereof (e.g. those salts which are in common usage) and other such pharmaceutically active ingredients described in European Pharmacopoeia, 3^{rd} Edition and one, two or more of them in combination.

In one embodiment of the invention the API is not acebutolol HCl, fluoxetine HCl, paracetamol, sodium valproate, ketoprofen or metformin HCl.

The method of the present invention may also be applicable to producing granules and tablets of the invention from material comprising further specific APIs, for example ibuprofen e.g. as sodium or sodium monohydrate, alclometasone dipropionate, allopurinol, alprazolam, amcinonide, amitriptyline e.g. as HCl, amoxicillin, atenolol, atracurium e.g. as besylate, azithromycin, aztreonam, beclomethasone, beclomethasone dipropionate, betamethasone, betamethasone acetate, betamethasone buteprate, betamethasone dipropionate, betamethasone disodium phosphate, betamethasone valerate, bivalirudin, bleomycin e.g. as sulfate, bortezomib, bromocriptine e.g. as mesilate, budesonide, buprenorphine e.g. as hydrochloride, butorphanol e.g. as tartrate, cabergoline, calcipotriene, calcitonin salmon, carbamazepine, carbidopa, carboplatin, carvedilol e.g. as phosphate, cefadroxil, cefdinir, cefprozil, cephalexin, chlormadinone e.g. as acetate, cilostazol, cisplatin, clarithromycin, clobetasol e.g. as propionate, clobetasone butyrate, clomiphene e.g. as citrate, clomipramine e.g. as HCl, clonazepam, clopidogrel e.g. as HBr, cyproterone e.g. as acetate, darifenacin, daunorubicin e.g. as HCl, deferasirox, deferoxamine e.g. as mesilate, deflazacort, deprodone propionate, desmopressin e.g. as acetate, desonide, desoximetasone, diazoxide, dicloxacillin, diflorasone e.g. as diacetate, difluprednate, dihydro-a-ergocriptine e.g. as mesylate, dihydroergocristine e.g. as mesylate, dihydroergotamine e.g. as mesylate, dihydroergotoxine e.g. as mesylate, diltiazem e.g. as HCl, docetaxel, dorzolamide e.g. as HCl, doxepin e.g. as HCl, doxorubicin e.g. as HCl, epirubicin e.g. as HCl, eptifibatide, ergometrine e.g. as maleate, ergotamine e.g. as tartrate, etodolac, etoposide, famciclovir, fludarabine e.g. as phosphate, fludrocortisone acetate, flumethasone, flumethasone e.g. as pivalate, flunisolide e.g. as anhydrous, fluocinolone e.g. as acetonide, fluocinonide, fluorometholone, fluticasone e.g. as propionate, fluvoxamine e.g. as maleate, formoterol e.g. as fumarate, fulvestrant, furosemide, gabapentin, galantamine e.g. as HBr, gemcitabine e.g. as HCl, gemfibrozil, halcinonide, haloperidol, haloperidol e.g. as decanoate, hydrochlorothiazide, idarubicin e.g. as HCl, imatinib, imipramine e.g. as HCl, imiquimod, indomethacin, labetalol e.g. as HCl, latanoprost, leflunomide, leuprolide/leuprorelin e.g. as acetate, levodopa, lisinopril, lisuride e.g. as maleate, loperamide, lovastatin, medroxyprogesterone acetate, megestrol e.g. as acetate, memantine, metaxalone, metergoline, methyldopa, methylergometrine meleate, methylprednisolone, metoprolol succinate, metoprolol e.g. as tartrate, mirtazapine, mitomycin, mitoxantrone e.g. as HCl, mometasone furoate, mupirocin, mupirocin e.g. as calcium, nabumetone, naproxen e.g. as sodium, nefazodone e.g. as HCl, nicergoline, norelgestromin, octreotide e.g. as acetate, olanzapine, olmesartan e.g. as medoxomil, ondansetron e.g. as HCl, oxaliplatin, oxazepam, paclitaxel, pancuronium e.g. as bromide, pantoprazole e.g. as sodium sesquihydrate, paramethasone acetate, paroxetine e.g. as HCl, pemetrexed diacid, pentoxifylline, pergolide e.g. as mesilate, pioglitazone e.g. as HCl, probenecid, prostaglandin, rocuronium e.g. as bromide, rosuvastatin e.g. as calcium, salbutamol (albuterol) e.g. as sulfate, sertraline e.g. as HCl, sildenafil, silymarine, solifenacin, tamoxifen e.g. as citrate, telmisartan, terazosin e.g. as HCl, terguride, teriparatide, ticlopidine e.g. as HCl, timolol e.g. as maleate, tobramycin, tobramycin e.g. as sulfate, torsemide, trazodone, triamcinolone, triamcinolone acetonide, trimethoprim, trimipramine e.g. as maleate, valaciclovir e.g. as HCl, vecuronium e.g. as bromide, venlafaxine e.g. as HCl, verapamil, zaleplon, zoledronic acid, zolpidem and zonisamide or a (or an alternative) pharmaceutically acceptable salt thereof, e.g. the HCl salt.

In one embodiment the API is not ibuprofen sodium or a hydrate thereof. In one embodiment the API is not ibuprofen sodium monohydrate. In another embodiment the API is not ibuprofen sodium dihydrate.

The method of the present invention may also be applicable to producing granules and tablets of the invention from material comprising further specific APIs, for example lamotrigine, ondansetron e.g. as hydrochloride, lamivudine, valacyclovir e.g. as hydrochloride, paroxetine e.g. as hydrochloride, zidovudine, carvedilol, ,rosiglitazone e.g. as maleate, abacavir e.g. as sulfate, bupropion e.g. as hydrochloride, topiramate, rabeprazole e.g. as sodium, galantamine e.g. as hydrobromide, risperidone, oxybutynin e.g. as chloride, repaglinide, venlafaxine e.g. as hydrochloride, ramipril, pravastatin e.g. as sodium, aripiprazole, efavirenz, levofloxacin, escitalopram e.g. as oxalate, memantine e.g. as hydrochloride, tenofovir, disoproxil e.g. as fumarate, simvastatin, alendronate e.g. as sodium, losartan e.g. as potassium, montelukast e.g. as sodium, finasteride, ezetimibe, rizatriptan e.g. as benzoate, mycophenolate mofetil, capecitabine, granisetron e.g. as hydrochloride, ritonavir, fenofibrate, bosentan, modafinil, clopidogrel e.g. as bisulfate, irbesartan, irbesartan-hydrochlorothiazide, drospirenone, desloratadine, lansoprazole, levetiracetam, quetiapine e.g. as fumarate, anastrozole, bicalutamide, candesartan cilexetil, zolmitriptan and sumatriptan e.g. as succinate or a (or an alternative) pharmaceutically acceptable salt thereof, e.g. the HCl salt.

The method of the present invention may also be applicable to producing granules and tablets of the invention from material comprising further specific APIs, for example atorvastatin e.g. as calcium, amlodipine e.g. as besylate, raloxifene e.g. as hydrochloride, tadalafil, pioglitazone e.g. as hydrochloride, duloxetine e.g. as hydrochloride, atomoxetine e.g. as hydrochloride, terbinafine e.g. as hydrochloride, benazepril e.g. as hydrochloride, letrozole, cyclosporine, rivastigmine e.g. as tartrate, fluvastatin e.g. as sodium, celecoxib, cetirizine e.g. as hydrochloride, tolterodine e.g. as tartrate, voriconazole, eletriptan e.g. as hydrobromide, pregabalin, sunitinib e.g. as malate and ziprasidone e.g. as hydrochloride or a (or an alternative) pharmaceutically acceptable salt thereof, e.g. the HCl salt.

The method of the present invention may also be applicable to producing granules and tablets of the invention from material comprising further specific APIs, for example 4-quinolinecarboxamide, 8-aminoquinoline, acyclovir, ALTU-135, apixaban, armodafinil, arzoxifene, asenapine, asimadoline, asoprisnil, bazedoxifene, belatacept, bendamustine e.g. as HCl, bifeprunox, binodenoson, brecanavir, brivaracetam, buprenorphine, canertinib, casopitant e.g. as mesylate, certolizumab pegol, cladribine, clazosentan, clevudine, clodronate, conjugated estrogens synthetic B, cyproterone, cytofab, dalbavancin, dapoxetine, darapladib, dasatinib, denagliptin, dienogest, doxepin, dronedarone, eculizumab, eltrombopag, elzasonan, enalapril, enzastaurin, eplivanserin, etaquine, ethynylcytidine, exenatide, farglitazar, gaboxadol, garenoxacin, gemcabene, glimepiride, combination of glimepiride and rosiglitazone, hydrocodone e.g. as bitartrate, combination of hydrocodone bitartrate and ibuprofen, indiplon, ixabepilone, lapatinib, lecozotan, lonafarnib, lorazepam, lubiprostone, lurasidone, maraviroc, merimepodib, mesalamine, mesopram, minodronate, motivizumab, muraglitazar, nalmefene, naltrexone, naveglitazar, odiparcil, ONO-2506, ONO-8025, orexin-RA-1, oxycodone, pazopanib, pertuzumab, pexelizumab, pleconaril, polyphenon E, posaconazole, prasugrel, pruvanserin, ribavirin, rimonabant, roflumilast, roflumilast, ruboxistaurin e.g. as mesylate, saredutant, satraplatin, saxagliptin, seletracetam, silodosin, sitafloxacin, sitagliptin, solabegron, solifenacin e.g. as succinate, soraprazan, telbivudine, teriflunomide, tesaglitazar, ticlimumab, varenicline e.g. as tartrate, vicriviroc, vildagliptin, vinflunine, vorinostat, xaliprodene, sibutramine e.g. as hydrochloride, miglustat, tamsulosin e.g. as hydrochloride, esomeprazole e.g. as magnesium, stavudine, amprenavir, thalidomide, lenalidomide, emtricitabine, dutasteride, itraconazole, indinavir e.g. as sulfate, aprepitant, orlistat, ganciclovir, oseltamivir e.g. as phosphate, nifedipine, temozolomide and dextroamphetamine e.g. as sulfate or a (or an alternative) pharmaceutically acceptable salt thereof, e.g. the HCl salt.

The method of the present invention may also be applicable to producing tablets of the invention from material comprising excipients or other ingredients usable in e.g. pharmaceutical industry, such as for example 2,6-dibutyl-4-methylphenol, 2-pyrrolidone, acacia powder, acesulfame potassium, acetyltributyl citrate, agar, albumin, alfa-starch, alginate, alginic acid, aliphatic polyesters, alitame, alpha tocopherol, aluminum hydroxide adjuvant, aluminum monostearate, aluminum oxide, aluminum phosphate adjuvant, aluminum stearate, ammonia solution, ammonium alginate, anhydrous calcium hydrogenphosphate, anhydrous calcium phosphate, anhydrous lactose, ascorbic acid, ascorbyl palmitate, aspartame, attapulgite, bentonite, benzalkonium chloride, benzoic acid, benzyl benzoate, boric acid, bronopol, butylated hydroxyanisole, butylparaben, calcium alginate, calcium carbonate, calcium lactate, calcium silicate, calcium stearate, calcium sulfate, carbomer, carboxymethyl cellulose, carboxymethylcellulose calcium, carboxymethylcellulose sodium, carrageenan, casein, cellulose acetate, cellulose acetate phthalate, ceratonia, cetrimide, cetylpyridinium chloride, chitosan, chlorhexidine, chlorocresol, chloroxylenol, cholesterol, cinnamon powder, citric acid monohydrate, citric acid, clay, colloidal silicon dioxide, coloring agents, copovidone, cresol, croscarmellose sodium, crospovidone, crystalline cellulose, crystalline cellulose-carmellose sodium, cyclodextrins, cyclomethicone, denatonium benzoate, dextrates, dextrin, dextrose, dibasic anhydrous calcium phosphate, dibasic dihydrate calcium phosphate, dibasic sodium phosphate, dibutyl phthalate, dibutyl sebacate, diethanolamine, diethyl phthalate, dihydroxyaluminum aminoacetate, dimethicone, dimethyl phthalate, dimethyl sulfoxide, dimethylacetamide, dimethylpolysiloxane, disodium edta, dl-malic acid, d-mannitol, docusate sodium, dry yeast, d-sorbitol, edetic acid, erythorbic acid, erythritol, ethyl acetate, ethyl lactate, ethyl maltol, ethyl oleate, ethyl vanillin, ethylcellulose, ethylene glycol palmitostearate, ethylene vinyl acetate copolymer,ethylparaben, fructose, fumaric acid, gelatin, glacial acetic acid, glucose, glycerin, glyceryl behenate, glyceryl monooleate, glyceryl monostearate, glyceryl palmitostearate, glycofurol, hectorite, hexetidine, hydrogenated castor oil, hydrogenated vegetable oil, hydrogenated cottonseed oil, hydrous lanolin, hydroxyethyl cellulose, hydroxyethylmethyl cellulose, hydroxypropyl cellulose, hydroxypropyl starch, hypromellose, hypromellose acetate succinate, hypromellose phthalate, imidurea, inulin, iron oxides, isomalt, kaolin, lactic acid, lactitol, lactose, lactose anhydrous, lactose monohydrate, lanolin, I-asparagic acid, lauric acid, lecithin, leucine, I-glutamine, linoleic acid, lowsubstituted hydroxypropyl cellulose, macrogol 15 hydroxystearate, magnesium aluminometasilicate, magnesium aluminum silicate, magnesium carbonate, magnesium hydroxide, magnesium oxide, magnesium silicate, magnesium stearate, magnesium trisilicate, maize starch, malic acid, maltitol, maltodextrin, maltol, maltose, mannitol, medicinal carbon, mediumchain triglycerides, meglumine, menthol, methylcellulose, methylcellulose phthalate, methylparaben, microcrystalline cellulose, monobasic sodium phosphate, monoethanolamine, monosodium glutamate, monothioglycerol, myristic acid, neohesperidin dc, nitrogen, nitrous oxide, nymcel™, octyldodecanol, paraffin, partially pregelatinized starch, pectin, petrolatum, petrolatum and lanolin alcohols, phenol, phenoxyethanol, phenylethyl alcohol, phenylmercuric acetate, phenylmercuric borate, phenylmercuric nitrate, phosphoric acid, polacrilin potassium, poloxamer, polycarbophil, polydextrose, polyethylene oxide, polymethacrylates, polymethyl vinyl ether maleic anhydride, polyoxyethylene stearates, polyvinyl acetate phthalate, polyvinylpyrrolidone, potassium acetate, potassium alginate, potassium benzoate, potassium bicarbonate, potassium chloride, potassium citrate, potassium hydrogen tartrate, potassium hydroxide, potassium metabisulfite, potassium sorbate, potato starch, powdered cellulose, powdered tragacanth, povidone, pregelatinized starch, propionic acid, propyl gallate, propylene carbonate, propylene glycol, propylene glycol alginate, propylparaben, pullulan, purified shellac, purified sucrose, raffinose, rice starch, saccharin, saccharin sodium, saponite, shellac, silicified microcrystalline cellulose, simethicone, skim milk powder, sodium acetate, sodium alginate, sodium ascorbate, sodium benzoate, sodium bicarbonate, sodium borate, sodium carboxymethyl cellulose, sodium carboxymethyl starch, sodium chloride, sodium citrate dihydrate, sodium cyclamate, sodium gluconate, sodium hyaluronate, sodium hydroxide, sodium lactate, sodium lauryl sulfate, sodium metabisulfite, sodium propionate, sodium starch glycolate, sodium stearyl fumarate, sodium sulfite, sorbic acid, sorbitan monostearate, sorbitol, spray dried lactose, starch, stearic acid, sterilizable maize starch, sucralose, sucrose, sugar spheres, sulfobutyl ether betacyclodextrin, sulfuric acid, synthetic aluminum silicate, talc, tartaric acid, thaumatin, thimerosal,thymol, titanium dioxide, tragacanth, trehalose, triacetin, tribasic calcium phosphate, tributyl citrate, triethanolamine, triethyl citrate, vanillin, wheat gluten powder, wheat starch, xylitol, zinc acetate and zinc stearate and possibly other such others classified as excipient in Arthur H. Kibbe: Handbook of Pharmaceutical Excipients, 3^{rd} Edition, and one, two or more of them in combination.

Examples of fluidizing agents include silicon compounds such as silicon dioxide hydrate, light silicic anhydride and others classified as fluidizing agents in Arthur H. Kibbe: Handbook of Pharmaceutical Excipients, 3rd Edition, and one, two or more of them in combination.

Granulate mass produced according to the method of the invention may have one or more of the following desirable properties: substantial absence of solid bridged between particles, good homogeneity, good flowability, good compressibility, good tabletability.

Granulate mass prepared according to the invention from a mixture of materials (eg with different particle sizes) surprisingly tends to be very homogeneous suggesting that the method of the invention is effective at countering any tendency for the materials to segregate (by contrast with what would be expected if fractionation were performed using sieves or other classifying means whose operation is based on fractionating material on the basis of particle size, for example).

Tablets produced according to the method of the invention may have one or more of the following desirable properties: good homogeneity, high tensile strength, fast disintegration time, need for only a low amount of lubricant.

Some embodiments of the invention are described herein, and further applications and adaptations of the invention will be apparent to those of ordinary skill in the art.

### BRIEF DESCRIPTION OF DRAWINGS

In the following, the invention is illustrated, but in no way limited by reference to the accompanying drawings in which
Fig. 1 show exemplary apparatus according to an embodiment of the invention,
Fig. 2a shows use of roller compactor according to an embodiment of the invention,
Fig. 2b shows use of roller compactor producing both avoidable dense (according to prior art) and desirable porous granules,
Fig. 2c shows an example of a granule produced by a method of prior art.
Fig. 2d shows an example of a granule produced according to processes of the present disclosure,
Fig. 2e shows yet another example of granules produced according to processes of the present disclosure,
Fig. 2f illustrates an example about formation of granular mass produced according to processes of the present disclosure, Fig 2g shows particle size distribution diagrams of materials shown in figure 2f,
Fig. 2h shows surface images of granules produced using different compaction forces according to processes of the present disclosure,
Fig. 3 shows an exemplary fractionating device that contains a rotating device usable according to an embodiment of the invention,
Fig. 4a and Fig. 4b show two alternative exemplary cylindrical components that can be used in the fractionating device shown in figure 3,
Fig 4c shows an exemplary perforated steel sheet that may be used as part of a rotating device according to an embodiment of the present invention,
Fig. 5 shows an exemplary dual-filter arrangement for enabling continuous operation of the system of an embodiment of the present invention,
Fig. 6 shows an exemplary arrangement for monitoring and adjusting the characteristics of the accepted granules in real time,
Fig. 7 shows an exemplary arrangement for mixing granulate masses from separately compacted substances, and
Fig. 8 shows an exemplary device for determining flowability of a powder or granulate mass.

### DETAILED DESCRIPTION OF DRAWINGS

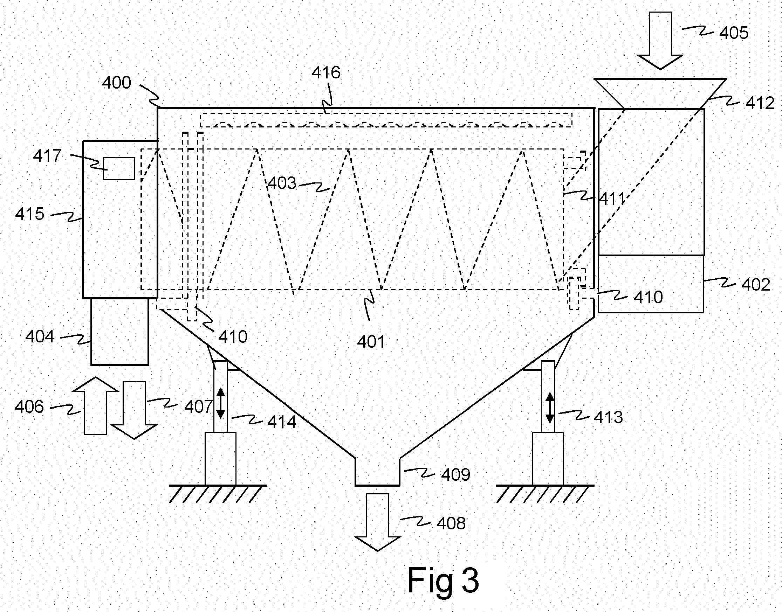

The apparatus 100 (figure 1) of an embodiment of the invention comprises a compacting device that compacts powder material into granules and a fractionating device that fractionates at least some fine particles and/or small granules away from acceptable granules. The fractionating device 112 in figure 1 is shown in more detail in figure 3. The apparatus shown in fig. 1 comprise a raw material feeding container 101, into which material to be granulated is fed. The feeding container is connected to a pneumatic conveyor pipeline 102, to which the material is passed through a feeder valve 103. The tubes of the pneumatic conveyor system have a diameter of about 47mm and their material may be for example some suitable plastic material, e.g. polyethene. The feeder valve may be a so-called star-shape flap valve. One such valve is manufactured by Italian pharmaceutical device manufacturer CO.RA™ (Lucca, Italy). In operation, the closing element of the valve may be turned 180° alternately in either direction, whereby buildup of the powder substance in the container can be avoided. Other equipment intended for continuous charging of powder substance, such as compartment feeders, may also be used.

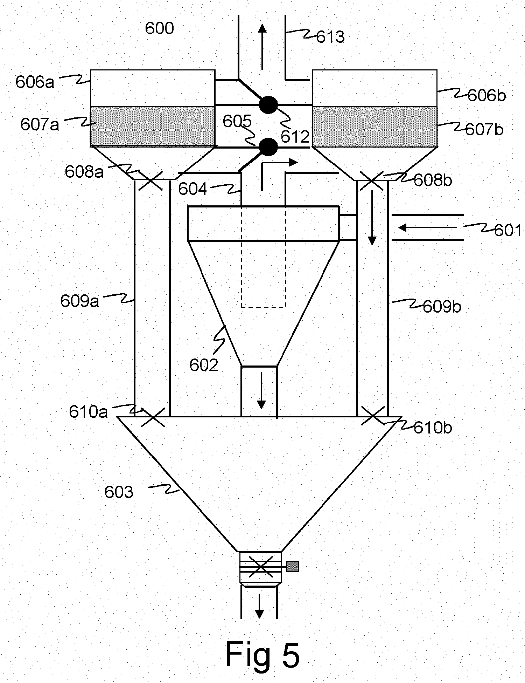

The pressure of the air flowing within the conveyor 102 may be adjusted to be lower than that of the surroundings. This may be achieved for example using an extractor suction fan 104. The suction fan is of make BUSCH™ (Maulburg, Germany) and model Mink MM 1202 AV. The fan may be operated for example at 1860 RPM. Makeup carrier gas may be supplied through a connection 105. The material fed from the feeding container is transported through the conveyor 102 into a separating device 106, wherein fine rejected particles and new feed from container 101 are separated from the carrier gas. The fan can be provided with filters (shown in figure 5) situated beside the separating device. The device may be capable of continuous operation. One such device is a cyclone. After the separating step, the separated powder falls into an intermediate vessel 107.

The container 107 can be mounted on load cells 108 to measure the weight of the material. The intermediate vessel 107 is provided with valves 109a and 109b which may be of the same type as the feeding container valve 103. From the intermediate vessel 107, the powder is transferred to a compacting device, e.g. roller compactor 110 to produce a ribbon of compacted material which is then passed to a flake crushing screen 111 where granules are created by crushing the ribbon. In the context of this invention, compacting is considered as the step of the process that produces granules to be fractionated, regardless of whether a separate screen or milling device 111 is used or not. The compaction force of the compactor 110 may be adjusted by e.g. altering the feed rate of the powder substance, the rotating speed of the rolls of the roller compactor, the pressure applied to the rolls of the compactor device and/or the thickness of the resulting ribbon. The compaction force applied by the compactor may be adjusted to a low level to achieve the desired properties of the compacted mass, e.g. the porosity of the resulting granules and/or proportion of fine particles and/or small granules. The compactor and the flake crushing screen are devices well known to a person skilled in the art. After passing the compacting and flake crushing devices, the material is partially in the form of granules, but part of the material will still be in the form of fine particles and/or small granules. The maximum size of the granules as well as the mean size of the granules may be affected by, for example, the mesh size of the flake crushing screen. It should be noted, however, that size of a granule may increase as result of agglomeration in the fractionating and/or conveying steps of the process.

In some embodiments (not shown in figure), the apparatus 100 may comprise more than one compacting device, e.g. roller compactor, to improve e.g. capacity and/or continuous processing capabilities of the apparatus. The compacting devices may require some periodic service breaks e.g. for cleaning up. The apparatus 100 may continue operation even if one of the compacting devices is being serviced.

The product from the above steps that contains fine particles and porous granules and that may be statically charged (e.g. by triboelectrification) is conveyed to a fractionating chamber 112.

There may be one or two e.g. star-shaped flap valves between compacting device and fractionating device to control the flow of compacted material to the fractionating device. The fractionating device divides the granulate mass into an accepted fraction and a rejected fraction on the basis of how different particles of the mass are affected by the carrier gas stream that flows in the fractionating device. The rejected fraction passes with the fed carrier gas stream to the feed conveyor 102, for re-processing, and the accepted fraction is led into a product container 113. By this means the product granules are treated gently and a relatively large volume of material comprising mostly fine particles and/or small granules is removed from the mass.

The operation of the fractionating chamber 112 is described in more detail with reference to Figures 3-5.

In the embodiments shown in Figure 1, load cells 108 are fitted to the container 107. Such sensors and other instrumentation can also be arranged in other containers and components of the system. Not all of the possible instrumentation is shown in the figure. For example the pneumatic conveyor, if required, may be provided with at least one pressure difference sensor 114, the information from which can be used to control the operation of the apparatus.

The present invention may also be carried out as a batch process where the reject fraction is not immediately returned to the system using the conveyor 102, but fed into a container of reject material. Such a system is not described in detail, but its construction and use will be readily apparent to those of skilled in the art.

The apparatus can be automated by transferring information received from the various sensors e.g. the pressure difference sensors 114, the load cells 108 and the valves 103 as well as information regarding the speed of rotation and the loads of the motors to a control unit and by applying appropriate control means 119. In some embodiments, the control means may monitor and control the amount of material currently in circulation in various components of the apparatus. For example, the control means may receive information from at least one of the load cells (scales) 108, 117, 118 of the apparatus and control operation of any of the valves 103, 109a and 109b according to the information received from the load cells. Further, the operation of suction fan 104 may be controlled e.g. according to information received from e.g. pressure difference sensor 114, from an instrument measuring gas flow rate or from any instrument measuring the properties, e.g. flowability, and/or amount of accepted granules.

In some embodiments where e.g. there is no control means 119, the valves of the process, e.g. 103, 109a and 109b, may be operated using timers that actuate a valve according e.g. to some suitable fixed or varying time interval.

Valves 109a and 109b may be operated so that flow of gas from the container 107 through the valves to the compacting device 110 is essentially prevented. For example, the valves 109a and 109b may be operated in an alternating manner so that at least one of the valves is kept closed at any given point of time during the operation of the apparatus 100. This way, the gas flow in the fractionating and conveying parts of the process is not disturbed.

For enhancing flow of powder material in the process, some vibrating or ultrasound emitting devices or other suitable means may be included e.g. in the components of the process where pneumatic conveying is not used. Such components may be e.g. the container 107, various parts of the compacting device 110 and flake crushing (granulator) device 111.

Control of the compaction force of the compacting device, e.g. roller compactor 110 is also useful, as granule structure as well as the proportion of fine particles and/or small granules is significantly affected by the compaction force used. The compaction force depends on a number of parameters, such as the rotating speed of the rolls and the feed rate of the powder substance. For example, the higher the feed rate of the powder substance for a given roller rotation rate, the higher the compaction force will be.

The exemplary apparatuses of figure 1 also comprise a receiver filter 115 and a safety filter 116. The receiver filter is the primary means of filtering any particles away from the gas that exits the system. However, as the materials processed by the system may be e.g. toxic or otherwise hazardous, a separate safety filter arrangement is required in many cases. There are multiple solutions known to a person skilled in the art that may be possible for the filtering arrangement 115 and 116. One receiver filter arrangement 115 suitable for e.g. an embodiment capable of continuous processing of powder material is described in figure 5.

The material of the conveyor 102 may be e.g. PVC, e.g. FDA PVC. Various components of the system may be connected together with electric wires for grounding purposes. Suitably the entire system is grounded.

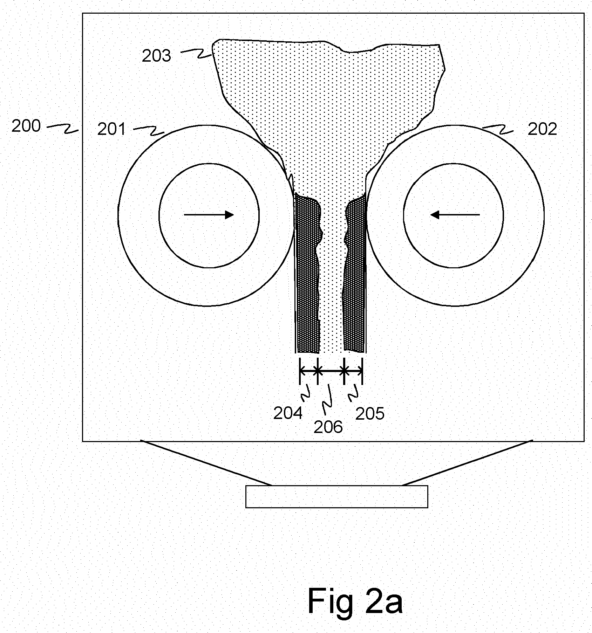

In figure 2a the roller compactor 200 compacts the mass 203 containing raw material and optionally particles recycled from the fractionating device into a ribbon 204, 205, 206 using rolls 201,202 that apply mechanical force to the mass to be compacted. Depending on the compaction force applied to the mass and the thickness of the ribbon, the amount of mass that gets compacted into granules 204, 205 varies. The remaining mass 206 may remain non-compacted or weakly compacted fine particles and/or weakly compacted small granules for example in the middle of the ribbon. The small granules and/or fine particles may not be capable of forming acceptable granules alone. However, the presence of such mass may have a positively contributing role in forming of acceptable granules in the fractionating and/or conveying steps of the process e.g. through triboelectrification and electrostatic forces. Depending on the feed material and compacting parameters, such as thickness of the ribbon, the proportion of fine particles and/or small granules may vary.

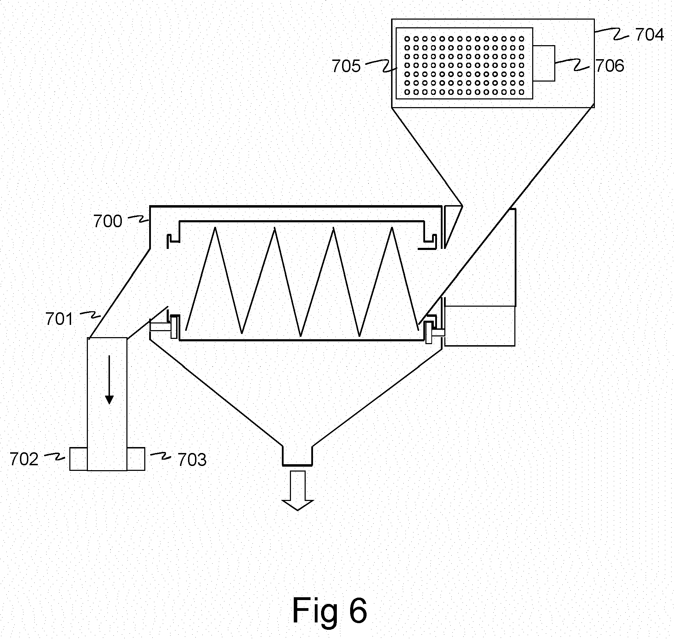

A convenient way to adjust operating parameters of the system is to set the compaction force of the roller compactor to the minimum that produces at least some well-flowing granules and set the rotating speed (see the description related to figure 3) of the fractionating device to the maximum available (e.g. about 100 RPM) in the device of make ROTAB™ (Donsmark Process Technology A/S, Denmark) and model 400EC/200 and then adjust the carrier gas flow rate so that acceptable granules with desired flow characteristics start flowing out the system. Too little gas flow in the fractionating device causes the proportion of fine particles and/or small granules to increase in the mass of accepted granules whereas use of too high a gas flow causes a large proportion of acceptable granules to be unnecessarily re-processed. Setup of the optimal gas flow may be done manually or automatically for example using real-time measurement of flow of accepted granules and characteristics of those granules. One such measurement arrangement is shown in figure 6.

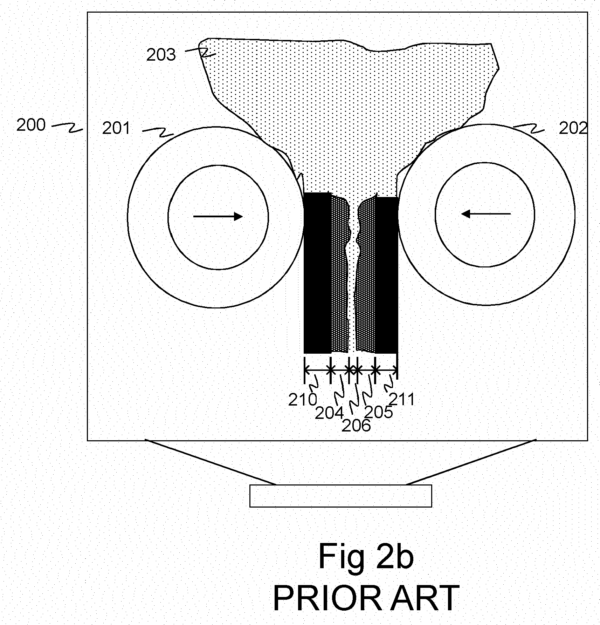

Figure 2b illustrates an example of the creation of unwanted dense granules and/or granules having solid bridges 210, 211 when a high compaction force as in the prior art is used. The more dense granules there are in the mass, the lower the quality of the mass may be for tableting. Although the flow characteristics of the mass resulting from using prior art high compaction forces (or repeated compaction with lower forces) may be acceptable even without fractionating, the compressibility and/or tabletability of the mass may with some materials be significantly lower, or some other characteristics of the tablet such as disintegration time may be undesirable. Moreover, significant heating of the material in the compaction step of prior art granulation process may be observed leading for example to formation of solid bridges through crystallization and/or degradation of components of the granules or undesirable characteristics of the granulate mass. Yet further, use of high compaction force typically reduces the proportion of small granules and/or fine particles 206 in the resulting granulate mass. Too low a percentage of such small granules and/or fine particles in the fractionating and/or conveying steps of the process may adversely affect the quality of the resulting accepted granules.

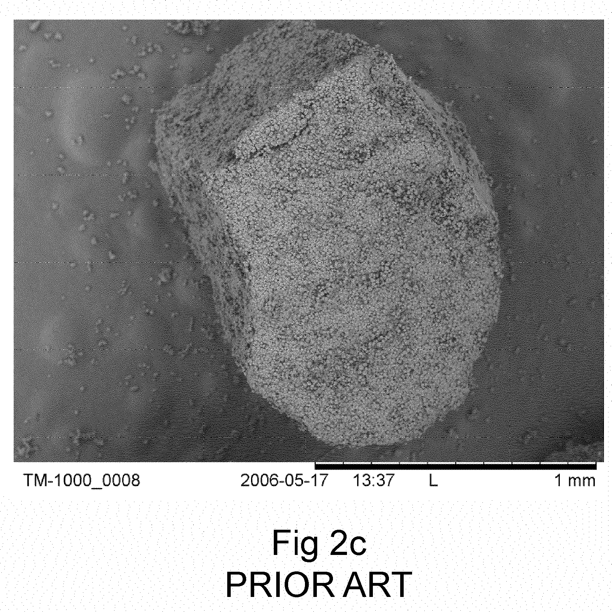

Figure 2c shows a scanning electronic microscope (SEM) picture of an exemplary dense maize starch granule that is produced using high compaction force (e.g. more than 80 kN using Hosokawa Bepex Pharmapaktor L200/50P roll compactor) for maize starch (CERESTAR™ product code C*Gel 03401, batch number SB4944) typical of the dry granulation methods of the prior art.

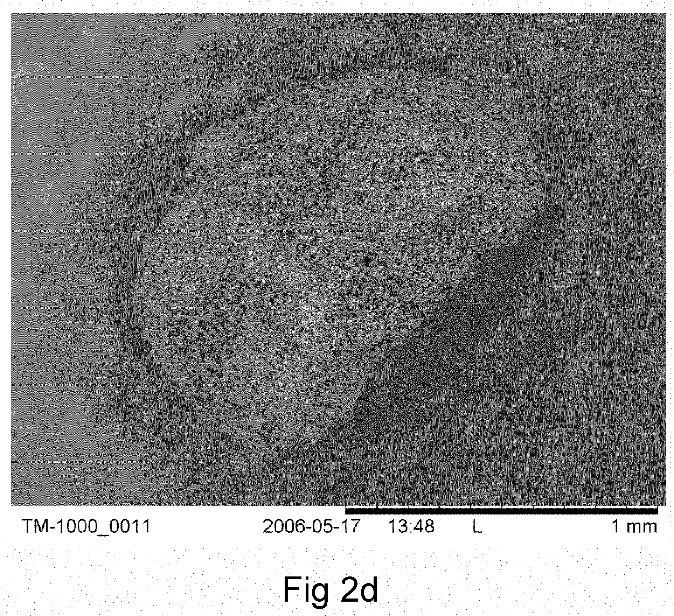

Figure 2d shows a picture of an exemplary porous starch granule of the same starch that is produced using low compaction force (in this case, 30-35 kN using the same Hosokawa roll compactor) and subsequent fractionation using gas stream. For different materials, the "low compaction force" that produces porous granules and "high compaction force" that produces unacceptable amount of dense granules and/or granules with solid bridges may vary. We have observed that the surface of the granule of Figure 2c is less porous (i.e more dense) than the granule of Figure 2d. There is more free space (i.e. pores) between the individual particles in the porous granule of Figure 2d than in the dense granule of Figure 2c. There also seems to be larger proportion of loosely attached particles on the surface of the porous granule of figure 2d than in the dense granule of figure 2d. Further, the granule of Figure 2c has more edges than the granule of figure 2d. The round shape of the porous granule may contribute to the good flow characteristics of the granulate mass containing such granules. The pores between particles on the surface of the porous granule as shown in Figure 2d may enhance the compressibility of the granule.

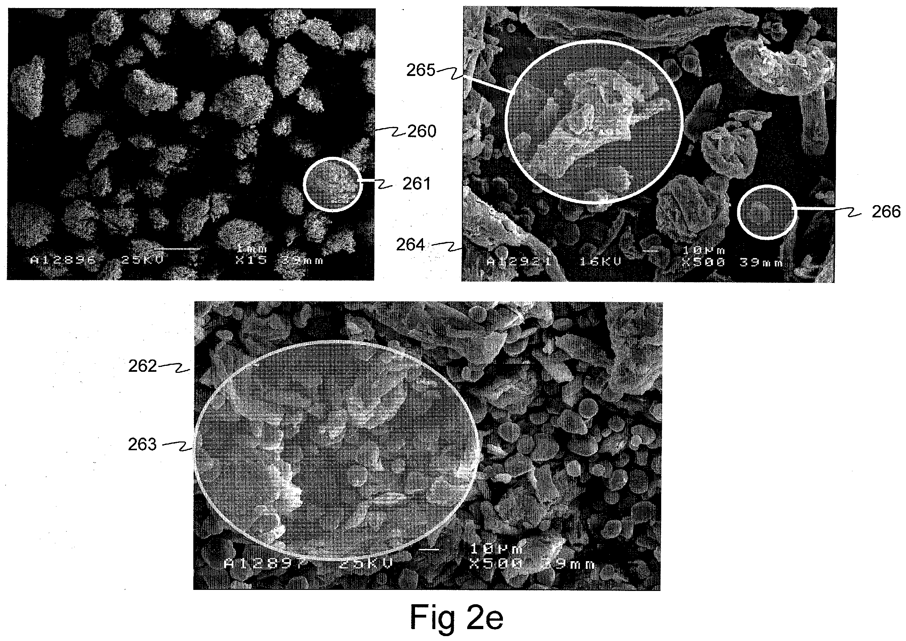

Figure 2e shows yet another embodiment of granules produced according to a process of the present disclosure. Image 260 shows a plurality of excipient granules 261 comprising 70% of microcrystalline cellulose and 30% of maize starch. A compaction force of 16kN was used in the granulation process. Typical size of a granule 261 in this sample is between 500 and 1000 µm. Image 262 shows a magnified picture of the surface of one of such granules. It may be observed from image 262 that the compacted surface of the granule is covered by small granules and/or fine particles 263 (e.g. in the range of ca 5 µm - 100 µm). Such individual small granules 265 and individual fine particles 266 are also shown in image 264. Small granules 265 and fine particles 266 are relatively loosely attached to the granule 261 forming a porous surface for the granule. The proportion of small granules (in this example, granules smaller than 106 µm) was approximately 20%. The flowability of the mass was observed to be excellent.

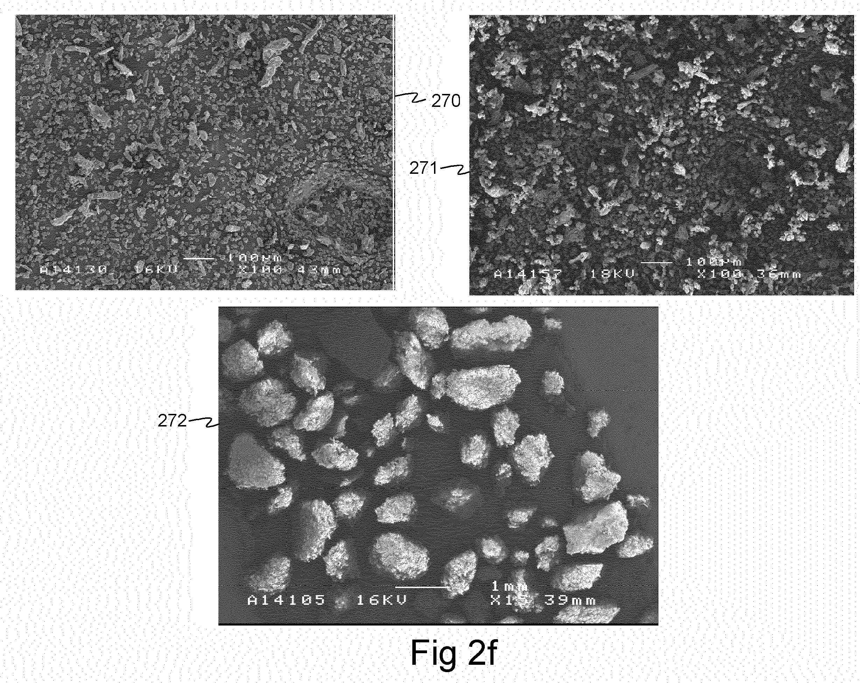

Figure 2f illustrates formation of granules from raw material comprising 50% microcrystalline cellulose and 50% of maize starch. Image 270 shows a SEM-image of unprocessed raw material. Image 271 shows a SEM-image of compacted but not yet fractionated granular mass. Compaction force of 25kN was used in the experiment. Image 272 shows a SEM-image of granular mass accepted by the fractionating device of an embodiment of the present invention. The magnification of images 270 and 271 is essentially similar and image 272 has 0.1x magnification in comparison to images 270 and 271. Image 270 shows practically no granules.

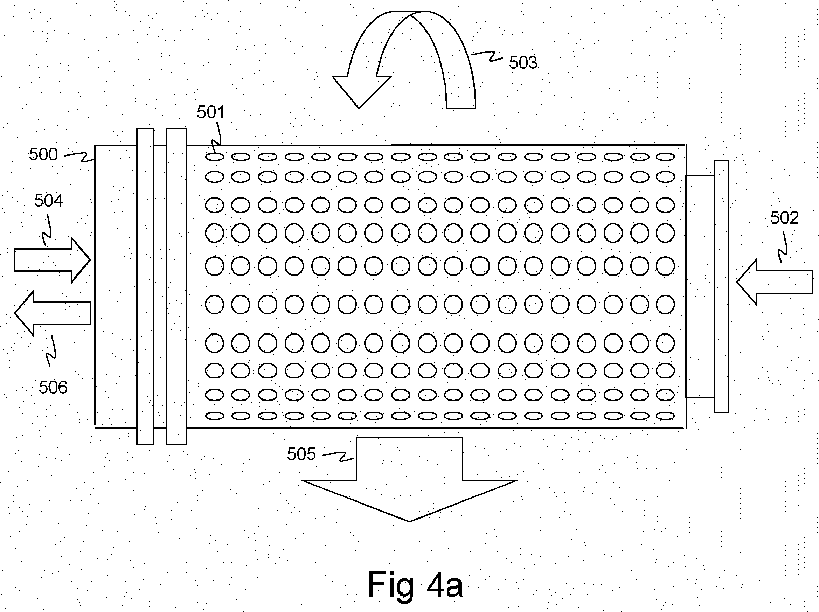

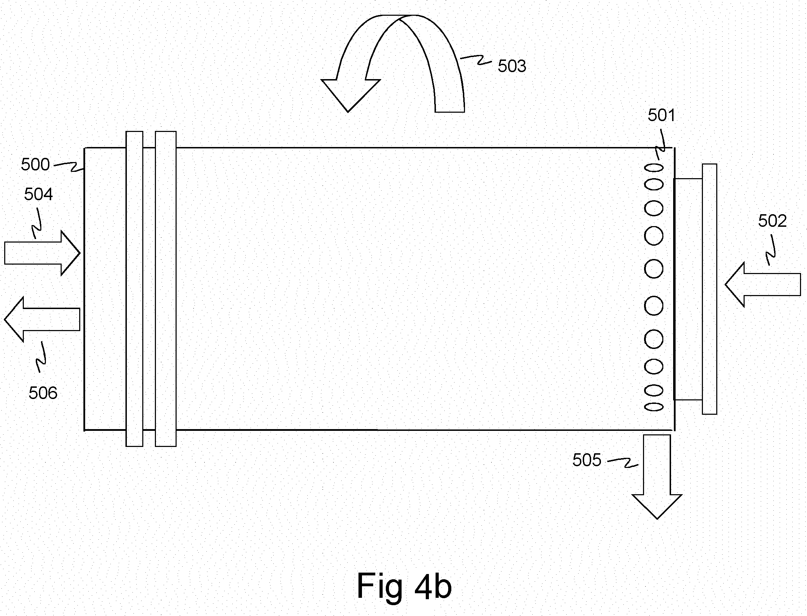

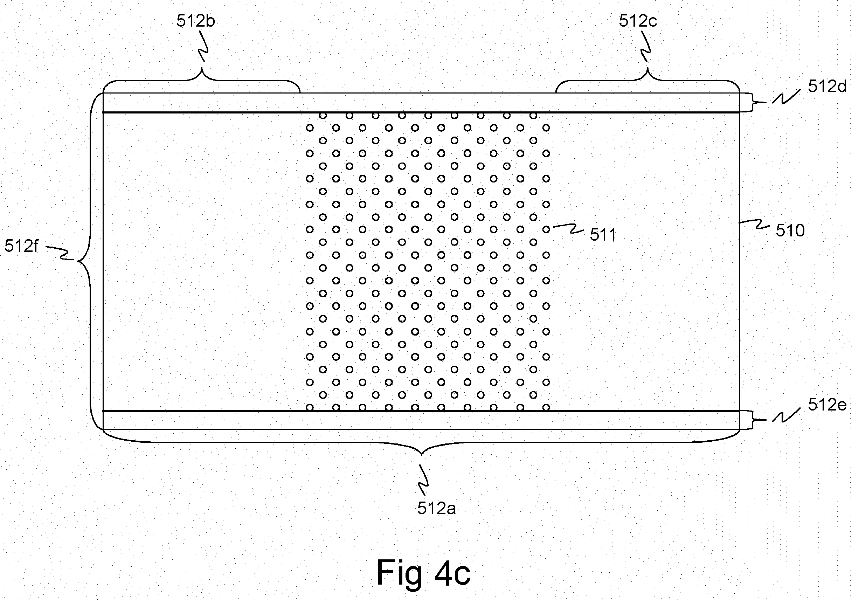

In image 271, attention is drawn to the relatively small size of the granules produced in the compacting step. Granules in the compacted mass 271 created by the roller compactor and flake crusher (110 and 111 in figure 1) are generally smaller than 500 µm whereas majority of the granules 272 accepted by the fractionating device (see figure 4) are larger than 500 µm. This surprising observation makes inventors believe that new acceptable granules may be created and/or granules may further agglomerate during the fractionating phase of the method of an embodiment of the present invention.

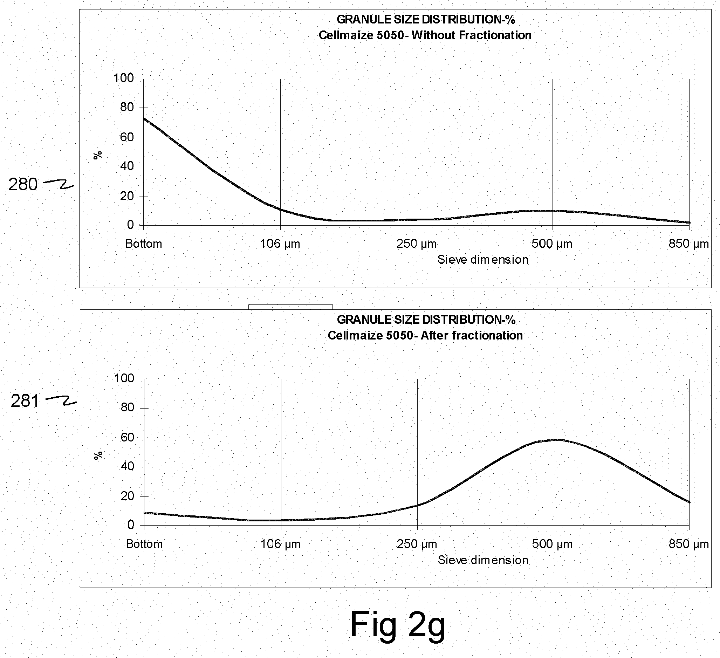

Figure 2g shows particle size distribution charts of materials depicted in images 271 and 272 of figure 2g. According to the product certification data of raw materials used, the size distribution of particles of the raw material (not shown in figures) is such that practically all particles of the mass are smaller than 106 µm. When the mass is compacted, the proportion of granules of acceptable size increases slightly as shown in image 280 but the majority (approximately 73%) of particles are still smaller than 106µm. Image 281 shows that after fractionation, the proportion of granules larger than 106µm increases significantly. The accepted fraction still contains about 10% of small granules and/or fine particles smaller than 106µm. Despite the relatively large proportion of small granules and/orfine particles, the mass exhibits excellent flowability. The total proportion of granules accepted from the compacted mass in the fractionating step was approximately 10%. Thus, approximately 90% of the mass was rejected by the fractionating device.

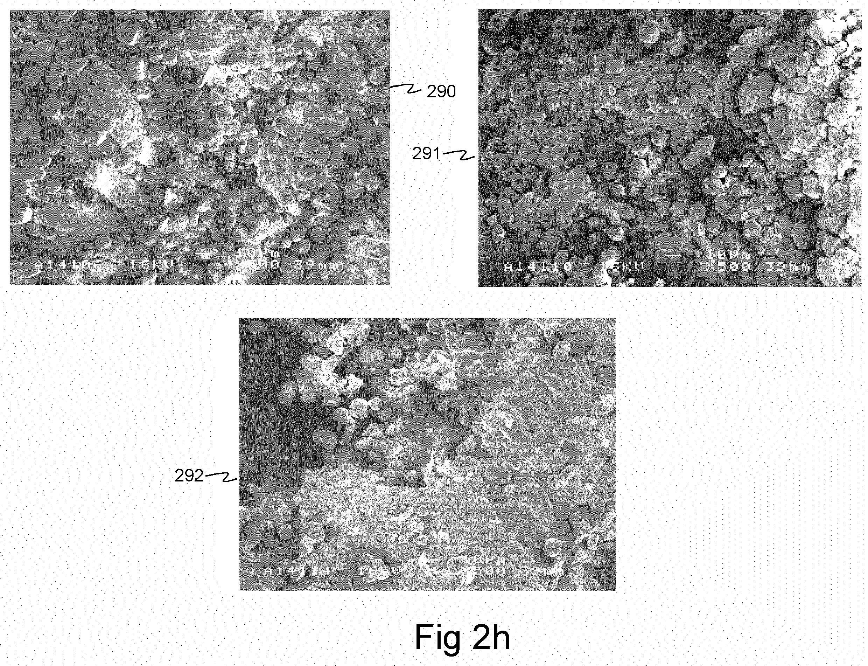

Figure 2h shows SEM-images of surfaces of granules manufactured using embodiments of the present disclosure. Different compaction forces have been used in the granulating process. The material shown comprises 50% of microcrystalline cellulose and 50% of maize starch. Images 290, 291, 292 depict granules produced using compaction force of 25kN, 40kN and 60kN, respectively. Attention is drawn to the decreasing surface porosity when the compaction force is increased. Numerous pores are easily detectable in granules of images 290 and 291 whereas there are large dense areas in granule of image 292. Lack of pores on the surface of the granule may deteriorate at least some of the properties of the granular mass, e.g. flowability of the mass, tablettability of the mass and/or disintegration time of resulting tablet. Thus it is suggested that the optimal compaction force for producing granules from this raw material is probably below 60kN. Although the SEM images 290, 291 do not show significant differences in the structure of the surface of the granule, the granular mass produced using compaction force of 25kN form tablets with higher tensile strength and quicker disintegration time than the mass produced with compaction force of 40kN.

Figure 3 illustrates an example of an enhanced fractionating device. In the figure, components and structures residing inside the device are drawn using dotted lines. The device 400 comprises a fractionating chamber and, mounted inside the chamber, an open ended cylinder (or cone-shaped device, not illustrated) 401 rotatably supported on rollers 410. The rotating speed of the cylinder can be adjusted to be for example the maximum available in the device of make ROTAB™ (Donsmark Process Technology A/S, Frederiksberg, Denmark) and model 400EC/200. The jacket of the cylinder or cone may be perforated. There are no restrictions with regard to the number and shape of the possible apertures or their edges except for that the apertures should be constructed so that the gas (air) together with entrained fine particles is able to leave the cylinder through them. The apertures may be, for instance, round, oval or slots. In one embodiment, the apertures are round and they have been cut using laser cutting techniques. In one embodiment, the diameter of the round apertures is 1.5mm. A drive motor 402 is arranged to rotate the cylinder at a suitable speed, e.g. at 100 RPM. A spiral structure 403 is provided inside the cylinder for transporting the solid material from the feed end 411 to the outlet 404 as the cylinder rotates. Instead of a spiral, various kinds of fins or other structures can be provided internally within the cylinder to obtain movement of the compacted material, and its interaction with the gas stream. The angle of inclination of the cylinder may be adjusted as required by, for instance, changing the position of the whole fractionating device 400 in its suspension structure 413,414.

The powder 405 leaving the compacting device falls through a charge connection 412 into the feed end 411 of the cylinder and is transported by the spiral 403 towards an outlet tube 404. The carrier gas 406 flowing through the outlet 404 moves in the opposite direction to the accepted granules 407. Acceptable granules pass along in the cylinder 401, and fall through the outlet 404 to a product container (not shown) by effect of gravitation. Unacceptable fine particles and/or small granules that may be accompanying the acceptable granules to the tube 404 are generally conveyed back from the tube 404 to the cylinder 401 by the gas stream 406. In the present device, the outlet 404 is a downward pointing tube whose length is 70 mm and diameter is 40 mm. The rejected fraction of fine particles and/or small granules 408 together with the carrier gas stream flows to the feeding conveyor (see 102 in figure 1), through connection 409 for reprocessing. The granules may grow in size in the fractionating device 400. This agglomeration may be caused e.g. by triboelectrification and electrostatic forces.

It is also noteworthy to observe that the cylinder 401 may act not only as a fractionating means but also as a buffer and conveyor of input material. Thus, this embodiment may provide benefits over the other fractionating means described herein. One such benefit is for example the ability to absorb bursts of input material 405 coming from the compacting device.

The embodiment shown in figure 3 comprises also means 416 for keeping the rotating cylinder clean. One such means blows pressurized gas (e.g. air) through a plurality of holes towards the cylinder 401. The pressure used may be e.g. 1-4 bar.

The fractionating means may also comprise means 417 for monitoring the progress of material in the fractionating device. Such means may be e.g. a sensor measuring the rotating speed of the cylinder or any other suitable means known to a person skilled in the art.

The properties of the accepted fraction may be influenced e.g. by changing the rotation speed of the cylinder, the angle of inclination of the cylinder, the pitch of the spiral, and the size, number and location and the shape of the apertures in the cylinder as well as by varying the flow rate of the carrier gas.

Figures 4a and 4b show two different forms of the cylinder-shaped device residing inside the fractionating device (see 400 in figure 3). A cylinder 500 has apertures 501 that in the figure 4a are situated throughout the jacket of the cylinder whereas in figure 4b there are apertures only in one end of the cylinder. The input material 502 that contains both granules and fine particles is fed to the rotating cylinder from one end of the cylinder. The rotating movement 503 of the cylinder 500 and the spiral (see 403 in figure 3) inside the cylinder push the input material towards the other end of the cylinder. While the material is moving in the cylinder, carrier gas flow 504 separates the acceptable granules from the rejected fine particles and/or small granules 505 which are conveyed out of the cylinder through apertures 501 with the carrier gas flow. The accepted granules 506 are eventually pushed out of the cylinder by the spiral structure that resides inside the cylinder.

In the shown embodiment, the rotating device is a cylinder of diameter of 190 mm and length of 516 mm and comprises apertures each having a diameter of 1.5 mm and the apertures reside on average 6 mm from each other. The air stream that enters the fractionating device through aperture 404 (figure 3) is further led out of the fractioning chamber for reprocessing through an aperture (409 in figure 3) of 50 mm in diameter. Inside the cylinder there is a screw-shaped guiding structure that advances 80 mm per revolution towards the aperture of accepted material 506. The height of the guiding structure is 25 millimeters. Figure 4c shows a drawing of an exemplary perforated 511 stainless steel sheet 510 that may be used to build a suitable cylinder. The thickness of the sheet is about 0.8mm. In this example sheet, dimension 512a is 51cm, dimensions 512b and 512c are 8 cm, dimensions 512d and 512e are 1 cm and dimension 512f is 48cm. The ROTAB™ device described above has been modified by changing the cylinder to one assembled from the steel sheet of figure 4c and the fractionating chamber has been changed to one having the shape similar to one shown in 400 of Figure 3.

Although the devices shown in Figures 4a and 4b are open-ended and cylinder shaped, and the movement involved is a rotating movement, conveyor devices of other shapes and utilizing other kinds of movements may also be used to convey the mass in the fractionating air stream.

The device may optionally be adapted to improve its continuous processing capabilities. One such adaptation is disclosed in figure 5 where a dual filter assembly is illustrated. The majority of fine particles and/or small granules is separated from carrier gas, e.g. air, in cyclone 602 (see also 106 in figure 1), but some fine particles and/or small granules may be sucked out of the cyclone with the carrier gas. Those particles may need to be filtered out before the carrier gas leaves the system. The filters 607a, 607b collect the fine particles and/or small granules until the filter is cleaned. One filter 607a, 607b may be active while the other is being cleaned e.g. by vibrating it. The valves 605, 612 may be used for guiding the gas flow through the active filter and for isolating the filter being cleaned from the gas stream. The powder resulting from the filter cleaning falls below the filter and further to a tube 609a, 609b when the valve 608a, 608b respectively is opened. In the other end of the tube, there may be a lower valve 610a, 610b that is opened after the upper valve 608a, 608b has been closed. Opening the lower valve causes the powder to fall back into the circulation for re-processing. This arrangement makes it possible to clean one of the filters while the apparatus is operational and the cleaning operation doesn't result in undesirable pressure shocks of carrier gas in the apparatus.

The apparatus may also optionally be equipped for example with sensors that measure e.g. the output rate of accepted material and/or size of accepted granules in real-time. An example about such an arrangement is shown in figure 6. Accepted granules leave the fractionating device 700 through tube 701. Light emitting devices 702 as well as light sensitive sensors 703 have been installed in the tube to observe the size of the passing accepted granules. Based on the information created by the sensors, the control logic of the system may adjust the operating parameters of the apparatus. One such adjustable parameter may be for example the size of granules produced by the flake crushing screen 704. Another such adjustable parameter may be the gas flow rate of the system. Yet further adjustable parameter is operation of any of the valves of the arrangement.

It may also be possible to equip the arrangement with a bulk flowability analyzer device that collects samples of accepted granules and tests their flowability, using e.g. a funnel illustrated in figure 8. Any operating parameter, e.g. gas flow rate, compaction force or rotating speed of the cylinder of the fractionating means may be adjusted if the accepted granules do not pass the flowability test.

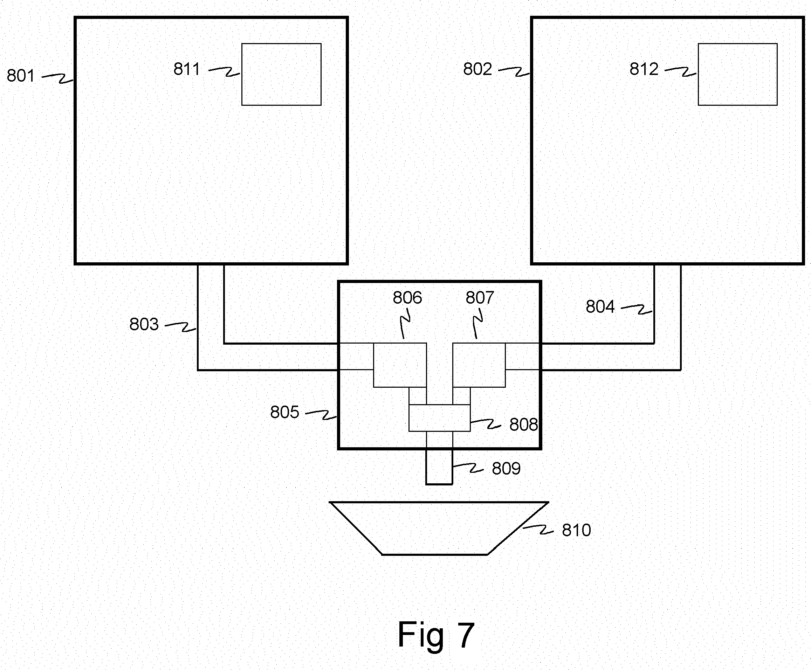

Figure 7 illustrates an exemplary optional arrangement for granulating powders separately and then mixing the granules together. The properties, e.g. disintegration time, of the end product, e.g. tablet, may be affected by granulating components of a formulation in multiple granulation processes vs. together in one process.

Granulation systems 801, 802 each produce granules from different substances (or from the same substance but with different granulation parameters such as compaction force or size of accepted granules). Each system has its own means 811, 812 of adjusting the granulation parameters. The accepted granules from each granulation system are led through a conveyor 803, 804 to a granule mixing device that has means 806, 807 to control the amount of each of the granules in the final mix. The mixing device may also have granule mixing means 808 to mix the granules together before the granulate mass flows to the container of final product 810 or directly to a tableting machine (not shown). The conveyor 803, 804 in figure 7 is a tube that leads to the mixing device, but the conveyor may also lead the granules into an intermediary storage container from which the mass may conveyed to the mixing device.

Figure 8 illustrates a simple device for measuring flowability of powder or granulate mass. Devices of different sizes are used for determining different degrees of flowability. The degree of flowability may be sufficient, good, very good or excellent.

The device for determining sufficient flowability has a smooth plastic surface cone 900 with a height 901 of 45 millimeters and with cone angle 902 of approximately 59 degrees and a round aperture 903 whose diameter is 12 millimeters. The length of tube 904 is 23mm. In a flowability test procedure, the cone is filled with powder or granulate mass while the round aperture 903 is kept closed. The aperture is opened, cone is knocked lightly to start the flow and the flow of the powder through the aperture by mere gravitation force is observed. Additional shaking or other kind of movement of the cone during the test is not allowed. The material passes the flowability test if the cone substantially empties. "Substantial" here means that at least 85%, 90% or 95% of the powder leaves the cone.

The device for determining good flowability using the test procedure explained above has a smooth glass surface cone 900 with a height 901 of 50 millimeters and with cone diameter 905 of 70mm and a round aperture 903 whose diameter is 7 millimeters. The length of tube 904 is 70 mm.

The device for determining very good flowability has a smooth plastic surface cone 900 with a height 901 of 35 millimeters and with cone diameter 905 of 48 mm and a round aperture 903 whose diameter is 4 millimeters. The length of tube 904 is 50 mm.

The device for determining excellent flowability has a smooth plastic surface cone 900 with a height 901 of 40 millimeters and with cone diameter 905 of 55 mm and a round aperture 903 whose diameter is 3 millimeters. The length of tube 904 is 60 mm.

Using the above mentioned or other embodiments of the present invention, it is possible to produce granules that have one or multiple of some desirable general characteristics, e.g. good flowability, good compressibility, good tablettability and quick disintegration time of a tablet. We have observed that those characteristics are applicable to many APIs and excipients. Thus, some potentially time-consuming and expensive parts of the drug formulation design process of prior art may be avoided with many APIs. The embodiments shown are also relatively cost-efficient to build and use. For example, it is possible to build an arrangement that is capable of producing several kilograms or tens of kilograms of granules per hour. The process is also relatively simple and easy to control in comparison to e.g. wet granulation methods of prior art. In the shown embodiments, there are few parameters that may need to be adjusted.

Percentage (%) values given herein are by weight unless otherwise stated.

Mean values are geometric mean values unless otherwise stated. Mean values of particle size are mean values based on weight.

To observe the characteristics of the granulate mass of various embodiments of the invention and their tabletability, sample mass and tablets may be produced according e.g. to following exemplary instructions.

The gas flow rate of the apparatus may be adjusted so that the fractionating effect of the gas flow resulted in a granulate mass that had good, very good or excellent flowability. For example, the gas flow rate may be achieved by operating the suction fan (BUSCH™ Mink MM 1202 AV) of the system at a default speed of approximately 1860 RPM. With some materials, the speed may need to be altered from the default to achieve desired quality of the granulate mass. The compaction force of the roller compactor may be adjusted to produce granules with optimal flow and tableting characteristics. The force used may be observed as kilonewtons as indicated by the roller compactor (HOSOKAWA Bepex Pharmapaktor L200/50P) used in the tests. The diameter of the rolls of the compactor may be e.g. 200mm and the working width of the rolls is 50mm. The thickness of the ribbon produced by the compactor may be about 4mm. The rotating speed of the rolls is typically between 10 and 12 RPM. The exact rotating speed is adjusted by the roller compactor to achieve the desired compaction force. The default mesh size of the flake crushing screen may be set e.g. to 1.00mm. In some experiments, the mesh size of the flake crushing screen may need to be altered from the default.

Unless specified differently, a rotating device as shown in figure 3 operating at about 100 RPM may be used as the fractionating means of the apparatus of the tests. The default size of apertures in the cylinder of the rotating means may be e.g. 1.5mm.

In tableting tests, 0.25% of magnesium stearate may be added to the granulate mass prior to tableting as a lubricant.

The particle size distribution of granulate mass may be measured e.g. using stack of sieves. In the measurements, the stack of four sieves may be shaken for 5 minutes using an Electromagnetic Sieve Shaker (manufacturer: C.I.S.A Cedaceria Industrial, S.L, model: RP 08) with power setting 6. The opening sizes of the sieves may be 850µm, 500µm, 250µm and 106µm.

To a person skilled in the art, the foregoing exemplary embodiments illustrate the model presented in this application whereby it is possible to design different granules and tablets, which in obvious ways utilize the inventive idea presented in this application.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

## Claims

1. A dry-granulation method for producing a tablet comprising (a) pharmaceutical active ingredient in an amount 50-90% w/w and (b) one or more excipients in an amount 10-50% w/w including at least a binder which comprises (i) preparing granules from a powder comprising a binder, a pharmaceutically active ingredient and optionally one or more other excipients or pharmaceutical active ingredients by a process in which a compaction force is applied to the powder to produce a compacted mass comprising a mixture of fine particles and granules and separating and removing fine particles and/or small granules from the granules by means of an apparatus comprising fractionating means wherein the fractionating means comprises a rotating device along the axis of which the compacted mass is moved in a gas stream through said rotating device, wherein the direction of the flow of the gas stream has a component which is contrary to that of the direction of flow of the compacted mass, wherein the rotating device comprises one or more apertures through which the gas stream flows out of the device and through which the fine particles and/or small granules are entrained and collecting the accepted granules; (ii) blending the accepted granules with other components of the tablet in granular or fine powder form wherein in step (ii) at least one other component of the tablet formulation is in granular form and is prepared from a powder comprising said other component by a process in which a compaction force is applied to the powder to produce a compacted mass comprising a mixture of fine particles and granules and separating and removing fine particles and/or small granules from the granules by means of an apparatus comprising fractionating means wherein the fractionating means comprises a rotating device along the axis of which the compacted mass is moved in a gas stream through said rotating device, wherein the direction of the flow of the gas stream has a component which is contrary to that of the direction of flow of the compacted mass, wherein the rotating device comprises one or more apertures through which the gas stream flows out of the device and through which the fine particles and/or small granules are entrained; and (iii) compressing the resultant blend to form a tablet; with the proviso that the tablet does not comprise (a) paracetamol, maize starch and microcrystalline cellulose in a ratio of 60:20:20 w/w, (b) acebutolol HCl and starch in a ratio of 90:10 w/w, (c) sodium valproate, hypromellose and maize starch in a ratio of 90:5:5 w/w, (d) ketoprofen and maize starch in a ratio of 50:50 w/w or (e) metformin HCl, microcrystalline cellulose and maize starch in a ratio of 80:14:6 w/w.

2. A method according to claim 1 wherein the rotating device is a cylinder or a cone.

3. A method according to claim 1 or claim 2 wherein the fractionating means does not require the passage of the compacted mass through any sieve.

4. A method according to any one of claims 1 to 3 wherein the direction of the flow of the gas stream is substantially contrary to that of the direction of the flow of the compacted mass.

5. A method according to any one of claims 1 to 4 wherein in step (i) granules are prepared from a powder also comprising a disintegrant.

6. A method according to any one of claims 1 to 5 wherein the binder is selected from synthetic polymers, saccharides, sugar alcohols, water-soluble polysaccharides, starches and inorganic compounds.

7. A method according to any one of claims 1 to 6 wherein the binder is selected from crospovidone, sucrose, glucose, lactose, fructose, mannitol, xylitol, maltitol, erythritol, sorbitol, crystalline cellulose, microcrystalline cellulose, powdered cellulose, hydroxypropylcellulose, methylcellulose, starches and polyvinylpyrrolidone and calcium carbonate.

8. A method according to claim 7 wherein the binder is microcrystalline cellulose.

9. A method according to any one of claims 1 to 8 wherein the disintegrant is selected from carboxymethyl cellulose, Nymcel™, sodium carboxymethyl cellulose, croscarmellose sodium, cellulose such as low substitution degree hydroxypropylcellulose, starch such as sodium carboxymethyl starch, hydroxypropyl starch, rice starch, wheat starch, potato starch, maize starch and partly pregelatinized starch.

10. A method according to claim 9 wherein the disintegrant is maize starch or carboxymethylcellulose.

11. A method according to any one of claims 1 to 10 wherein the tablet comprises (a) pharmaceutical active ingredient in an amount 70-90% w/w and (b) two or more excipients in an amount 10-30% w/w including at least a binder and a disintegrant.

12. A method according to any one of claims 1 to 11 wherein the ratio of binder to disintegrant is between 10:1 and 1:1 w/w.

13. A method according to any one of claims 1 to 12 wherein the tablet comprising (a) pharmaceutical active ingredient in an amount 51-89% w/w and (b) one or more excipients in an amount 11-49% w/w including at least a binder.

14. A method according to any one of claims 1 to 13 wherein the pharmaceutical active ingredient is not acebutolol HCl, fluoxetine HCl, paracetamol, sodium valproate, ketoprofen or metformin HCl.

15. A tablet obtained by the method of any one of claims 1 to 14.

## Patentansprüche

1. Trockengranulationsverfahren zur Herstellung einer Tablette, die (a) pharmazeutischen Wirkstoff in einer Menge von 50-90 Gew.-% und (b) einen oder mehrere Hilfsstoffe in einer Menge von 10-50 Gew.-%, einschließlich wenigstens eines Bindemittels, umfasst, umfassend (i) Herstellen von Granulatkörnern aus einem Pulver, das ein Bindemittel, einen pharmazeutischen Wirkstoff und gegebenenfalls einen oder mehrere andere Hilfsstoffe oder pharmazeutische Wirkstoffe umfasst, durch ein Verfahren, bei dem eine Kompaktierungskraft auf das Pulver ausgeübt wird, um eine kompaktierte Masse zu erzeugen, die ein Gemisch von feinen Partikeln und Granulatkörnern umfasst, und Abtrennen und Entfernen von feinen Partikeln und/oder kleinen Granulatkörnern von den Granulatkörnern mithilfe einer Vorrichtung, die eine Fraktionierungseinrichtung umfasst, wobei die Fraktionierungseinrichtung eine sich drehende Vorrichtung umfasst, entlang deren Achse die kompaktierte Masse in einem Gasstrom durch die sich drehende Vorrichtung bewegt wird, wobei die Flussrichtung des Gasstroms eine Komponente aufweist, die der Flussrichtung der kompaktierten Masse entgegengesetzt ist, wobei die sich drehende Vorrichtung eine oder mehrere Öffnungen umfasst, durch die der Gasstrom aus der Vorrichtung fließt und durch die die feinen Partikel und/oder kleinen Granulatkörner mitgerissen werden, und Sammeln der akzeptierten Granulatkörner; (ii) Mischen der akzeptierten Granulatkörner mit anderen Komponenten der Tablette in Granulatform oder feiner Pulverform, wobei bei Schritt (ii) wenigstens eine andere Komponente der Tablettenformulierung in Granulatform vorliegt und aus einem Pulver, das die andere Komponente enthält, durch ein Verfahren hergestellt ist, bei dem eine Kompaktierungskraft auf das Pulver ausgeübt wird, um eine kompaktierte Masse zu erzeugen, die ein Gemisch von feinen Partikeln und Granulatkörnern umfasst, und Abtrennen und Entfernen von feinen Partikeln und/oder kleinen Granulatkörnern von den Granulatkörnern mithilfe einer Vorrichtung, die eine Fraktionierungseinrichtung umfasst, wobei die Fraktionierungseinrichtung eine sich drehende Vorrichtung umfasst, entlang deren Achse die kompaktierte Masse in einem Gasstrom durch die sich drehende Vorrichtung bewegt wird, wobei die Flussrichtung des Gasstroms eine Komponente aufweist, die der Flussrichtung der kompaktierten Masse entgegengesetzt ist, wobei die sich drehende Vorrichtung eine oder mehrere Öffnungen umfasst, durch die der Gasstrom aus der Vorrichtung fließt und durch die die feinen Partikel und/oder kleinen Granulatkörner mitgerissen werden; und (iii) Komprimieren des erhaltenen Gemischs, um eine Tablette zu bilden; mit der Maßgabe, dass die Tablette nicht umfasst: (a) Paracetamol, Maisstärke und mikrokristalline Cellulose in einem Verhältnis von 60:20:20 Gew./Gew., (b) Acebutolol-HCl und Stärke in einem Verhältnis von 90:10 Gew./Gew., (c) Natriumvalproat, Hypromellose und Maisstärke in einem Verhältnis von 90:5:5 Gew./Gew., (d) Ketoprofen und Maisstärke in einem Verhältnis von 50:50 Gew./Gew. oder (e) Metformin-HCl, mikrokristalline Cellulose und Maisstärke in einem Verhältnis von 80:14:6 Gew./Gew.

2. Verfahren gemäß Anspruch 1, wobei die sich drehende Vorrichtung ein Zylinder oder ein Kegel ist.

3. Verfahren gemäß Anspruch 1 oder Anspruch 2, wobei die Fraktionierungseinrichtung nicht das Durchtreten der kompaktierten Masse durch ein Sieb erfordert.

4. Verfahren gemäß einem der Ansprüche 1 bis 3, wobei die Flussrichtung des Gasstroms der Flussrichtung der kompaktierten Masse im Wesentlichen entgegengesetzt ist.

5. Verfahren gemäß einem der Ansprüche 1 bis 4, wobei bei Schritt (i) Granulatkörner aus einem Pulver hergestellt werden, das auch ein Sprengmittel umfasst.

6. Verfahren gemäß einem der Ansprüche 1 bis 5, wobei das Bindemittel ausgewählt ist aus synthetischen Polymeren, Sacchariden, Zuckeralkoholen, wasserlöslichen Polysacchariden, Stärken und anorganischen Verbindungen.

7. Verfahren gemäß einem der Ansprüche 1 bis 6, wobei das Bindemittel ausgewählt ist aus Crospovidon, Saccharose, Glucose, Lactose, Fructose, Mannit, Xylit, Maltit, Erythrit, Sorbit, kristalliner Cellulose, mikrokristalliner Cellulose, pulverförmiger Cellulose, Hydroxypropylcellulose, Methylcellulose, Stärken und Polyvinylpyrrolidon und Calciumcarbonat.

8. Verfahren gemäß Anspruch 7, wobei das Bindemittel mikrokristalline Cellulose ist.

9. Verfahren gemäß einem der Ansprüche 1 bis 8, wobei das Sprengmittel ausgewählt ist aus Carboxymethylcellulose, Nymcel™, Natriumcarboxymethylcellulose, Croscarmellose-Natrium, Cellulose, wie z. B. Hydroxypropylcellulose mit niedrigem Substitutionsgrad, Stärke, wie z. B. Natriumcarboxymethylstärke, Hydroxypropylstärke, Reisstärke, Weizenstärke, Kartoffelstärke, Maisstärke und teilweise vorgelatinierte Stärke.

10. Verfahren gemäß Anspruch 9, wobei das Sprengmittel Maisstärke oder Carboxymethylcellulose ist.

11. Verfahren gemäß einem der Ansprüche 1 bis 10, wobei die Tablette umfasst: (a) pharmazeutischen Wirkstoff in einer Menge von 70-90 Gew.-% und (b) zwei oder mehr Hilfsstoffe in einer Menge von 10-30 Gew.-%, einschließlich wenigstens eines Bindemittels und eines Sprengmittels.

12. Verfahren gemäß einem der Ansprüche 1 bis 11, wobei das Verhältnis von Bindemittel zu Sprengmittel zwischen 10:1 und 1:1 Gew./Gew. beträgt.

13. Verfahren gemäß einem der Ansprüche 1 bis 12, wobei die Tablette umfasst: (a) pharmazeutischen Wirkstoff in einer Menge von 51-89 Gew.-% und (b) einen oder mehrere Hilfsstoffe in einer Menge von 11-49 Gew.-%, einschließlich wenigstens eines Bindemittels.

14. Verfahren gemäß einem der Ansprüche 1 bis 13, wobei der pharmazeutische Wirkstoff nicht Acebutolol-HCl, Fluoxetin-HCl, Paracetamol, Natriumvalproat, Ketoprofen oder Metformin-HCl ist.

15. Tablette, erhalten durch das Verfahren gemäß einem der Ansprüche 1 bis 14.

## Revendications

1. Procédé de granulation à sec pour la production d'un comprimé comprenant (a) un principe actif pharmaceutique à une teneur de 50 à 90 % en masse et (b) un ou plusieurs excipients à une teneur de 10 à 50 % en masse incluant au moins un agent liant qui comprend (i) la préparation de granules à partir d'une poudre comprenant un agent liant, un principe actif pharmaceutique et éventuellement un ou plusieurs autres excipients ou principes actifs pharmaceutiques par un procédé dans lequel une force de compactage est appliquée à la poudre pour produire une masse compactée comprenant un mélange de particules fines et de granules et la séparation et l'élimination des particules fines et/ou des petits granules d'avec les granules par le biais d'un appareil comprenant un moyen de fractionnement où le moyen de fractionnement comprend un dispositif rotatif le long de l'axe selon lequel la masse compactée est déplacée dans un flux gazeux à travers ledit dispositif rotatif, où la direction du débit du flux gazeux présente une composante qui est contraire à celle de la direction du débit de la masse compactée, où le dispositif rotatif comprend une ou plusieurs ouvertures à travers lesquelles le flux gazeux sort du dispositif et à travers lesquelles les particules fines et/ou les petits granules sont entraînés et la collecte des granulés acceptés ; (ii) le mélangeage des granulés acceptés avec les autres composants du comprimé sous forme granulaire ou de poudre fine où, dans l'étape (ii), au moins un autre composant de la formule de comprimé se présente sous forme granulaire et est préparé à partir d'une poudre comprenant ledit autre composant par un procédé dans lequel une force de compactage est appliquée à la poudre pour produire une masse compactée comprenant un mélange de particules fines et de granules et la séparation et l'élimination des particules fines et/ou des petits granules d'avec les granules par le biais d'un appareil comprenant un moyen de fractionnement, où le moyen de fractionnement comprend un dispositif rotatif le long de l'axe selon lequel la masse compactée est déplacée dans un flux gazeux à travers ledit dispositif rotatif, où la direction du débit du flux gazeux présent une composante qui est contraire à celle de la direction du débit de la masse compactée, où le dispositif rotatif comprend une ou plusieurs ouvertures à travers lesquelles le flux gazeux sort du dispositif et à travers lesquelles les particules fines et/ou les petits granules sont entraînés ; et (iii) la compression du mélange résultant pour former un comprimé ; à la condition que le comprimé ne comprenne pas (a) du paracétamol, de l'amidon de maïs et de la cellulose microcristalline dans un rapport de 60:20:20 en masse, (b) de l'acébutolol HCl et de l'amidon dans un rapport de 90:10 en masse, (c) du valproate de sodium, de l'hypromellose et de l'amidon de maïs dans un rapport de 90:5:5 en masse, (d) du kétoprofène et de l'amidon de maïs dans un rapport de 50:50 en masse ou (e) de la metformine HCl, de la cellulose microcristalline et de l'amidon de maïs dans un rapport de 80:14:6 en masse.

2. Procédé selon la revendication 1, où le dispositif rotatif est un cylindre ou un cône.

3. Procédé selon la revendication 1 ou la revendication 2, où le moyen de fractionnement n'exige pas le passage de la masse compactée à travers un quelconque tamis.

4. Procédé selon l'une quelconque des revendications 1 à 3, où la direction du débit du flux gazeux est substantiellement contraire à la direction du débit de la masse compactée.

5. Procédé selon l'une quelconque des revendications 1 à 4, où dans l'étape (i), les granules sont préparés à partir d'une poudre comprenant également un agent délitant.

6. Procédé selon l'une quelconque des revendications 1 à 5, où l'agent liant est choisi parmi les polymères de synthèse, les saccharides, les alcools sucrés, les polysaccharides hydrosolubles, les amidons et les composés inorganiques.

7. Procédé selon l'une quelconque revendications 1 à 6, où l'agent liant est choisi parmi les suivants : crospovidone, sucrose, glucose, lactose, fructose, mannitol, xylitol, maltitol, érythritol, sorbitol, cellulose cristalline, cellulose microcristalline, cellulose en poudre, hydroxypropylcellulose, méthylcellulose, amidons et polyvinylpyrrolidone et carbonate de calcium.

8. Procédé selon la revendication 7, où l'agent liant est la cellulose microcristalline.

9. Procédé selon l'une quelconque des revendications 1 à 8, où l'agent délitant est choisi parmi la carboxyméthylcellulose, la carboxyméthylcellulose sodium, Nymcel™, le croscarmellose sodium, la cellulose telle que l'hydroxypropylcellulose de faible degré de substitution, l'amidon tel que carboxyméthylamidon sodium, hydroxypropylamidon, amidon de riz, amidon de blé, amidon de pomme de terre, amidon de maïs et amidon partiellement prégélifié.

10. Procédé selon la revendication 9, où l'agent délitant est l'amidon de maïs ou la carboxyméthylcellulose.

11. Procédé selon l'une quelconque des revendications 1 à 10, où le comprimé comprend (a) un principe actif pharmaceutique à une teneur de 70 à 90 % en masse et (b) deux excipients ou plus à une teneur de 10 à 30 % en masse, incluant au moins un agent liant et un agent délitant.

12. Procédé selon l'une quelconque des revendications 1 à 11, où le rapport de l'agent liant sur l'agent délitant est compris entre 10:1 et 1:1 en masse.

13. Procédé selon l'une quelconque des revendications 1 à 12, où le comprimé comprend (a) un principe actif pharmaceutique à une teneur de 51 à 89 % en masse et (b) un ou plusieurs excipients à une teneur de 11 à 49 % en masse, incluant au moins un agent liant.

14. Procédé selon l'une quelconque des revendications 1 à 13, où le principe actif pharmaceutique n'est pas acébutolol HCl, fluoxétine HCl, paracétamol, valproate de sodium, kétoprofène ou metformine HCl.

15. Comprimé obtenu par le procédé selon l'une quelconque des revendications 1 à 14.
